(19) Europäisches Patentamt
European Patent Office
Office européen des brevets

(11) Publication number: **0 320 782 B1**

(12) **EUROPEAN PATENT SPECIFICATION**

(45) Date of publication of patent specification: **12.05.93**

(51) Int. Cl.[5]: **A01N 43/58**, A01N 37/50, C07C 249/16, C07C 251/80, C07D 237/28

(21) Application number: **88120477.0**

(22) Date of filing: **07.12.88**

The file contains technical information submitted after the application was filed and not included in this specification

# (54) Plant male sterilant.

(30) Priority: **18.12.87 JP 322040/87**
**18.12.87 JP 322041/87**

(43) Date of publication of application:
**21.06.89 Bulletin 89/25**

(45) Publication of the grant of the patent:
**12.05.93 Bulletin 93/19**

(84) Designated Contracting States:
**CH DE ES FR GB IT LI NL**

(56) References cited:
**EP-A- 0 138 661**
**EP-A- 0 363 236**

(73) Proprietor: **SUMITOMO CHEMICAL COMPANY, LIMITED**
**Kitahama 4-chome 5-33**
**Chuo-ku Osaka 541(JP)**

(72) Inventor: **Mizutani, Masato**
**2-10-5-301, Sonehigashi-machi**
**Toyonaka-shi Osaka-fu(JP)**
Inventor: **Shirosita, Masao**
**2-3-12, Miyahara Yodogawa-ku**
**Osaka-shi Osaka-fu(JP)**
Inventor: **Okuda, Hiroki**
**1-3-7-601, Shimae-cho**
**Toyonaka-shi Osaka-fu(JP)**
Inventor: **Mito, Nobuaki**
**2-14-7, Mefu**
**Takarazuka-shi Hyogo-ken(JP)**
Inventor: **Sakaki, Masaharu**
**2-10-3-329, Sonehigashi-machi**
**Toyonaka-shi Osaka-fu(JP)**

(74) Representative: **Türk, Gille, Hrabal, Leifert**
**Brucknerstrasse 20**
**W-4000 Düsseldorf 13 (DE)**

EP 0 320 782 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a plant male sterilant which comprises a compound of formula (I) or of formula (II) following below as an active ingredient, a method for inducing male sterility in a plant as well as new hydrazone derivatives per se having the formula (II).

In recent years, the food crisis has caused public discussion and the increase of producing food has become a big problem. Under this situation, the production of hybrid seeds has attracted attention.

It is known that the first filial generation plant has many outstanding characters such as an increased yield compared with its parent variety, owing to its vigorous growth. In order to obtain hybrid seeds it is necessary to prevent a self-pollination of a female parent and stamens of the female parent have to be removed for that purpose.

Hitherto, there have made a lot of efforts for the operation of removing stamens, i.e. castration, and also, since grains having a high rate of self-pollination, e.g. rice and wheat, have both stamens and pistils in small spikelet, it has been almost impossible to produce the hybrid seeds manually.

There are other methods such as a use of a cytoplasmic male sterility, but this method has problems such as it takes a long time for its breeding. Therefore, in recent years, it has been desired simple and sure methods to induce male sterility in plant without losing a pollination ability of the female parent.

Some kinds of cinnoline derivatives are described in some literature ["Zh. Obshch. Khim.", 37, 2487 (1967); "J. Chem. Soc. Chem. Commun.", 1974, 752; and "Synthesis", 1983, 52] and also disclosed in US-A- 4 604 134 and EP-A-0 197 226 as a chemical hybridizing agent.

However, the above mentioned compounds are not necessarily satisfactory since their efficiency is insufficient or they show a chemical injury on crop.

The same applies to the cinnoline derivatives described in EP-A-0 138 661 as plant male sterilants the sterility rates of which are insufficient as shown in Test Example 3 following below.

Therefore, the object of the present invention is to find a simple and sure means to induce male sterility in plant without losing a pollination ability of the female parent.

As the result of continuous efforts of the present inventors now it has been found that a compound having the formula (I) or the formula (II) following below can induce the male sterility in plant very simply and efficiently by treating plant with this compound.

Subject-matter of the present invention is a plant male sterilant, which comprises as an active ingredient an effective amount of a compound having the general formula (I):

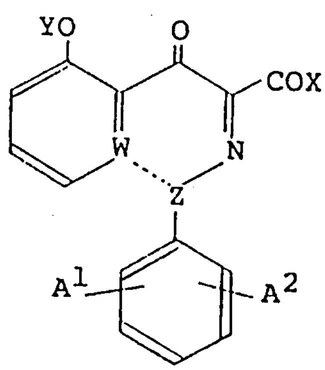

(I)

wherein:

X is $-OH$, $-O^{-}M^{+}$, $-OR^1$ or

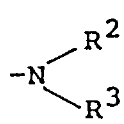

wherein $M^+$ is an alkali metal cation, an alkaline earth metal cation or

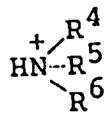 ,

in which $R^4$, $R^5$ and $R^6$ are the same or different and each is a hydrogen atom, a $C_1-C_6$-alkyl group, a $C_3-C_4$-alkenyl group, a $C_3-C_4$-alkynyl group, a $C_3-C_8$-cycloalkyl group, a benzyl group or a phenyl group; $R^1$ is a $C_1-C_9$-alkyl group, a $C_3-C_6$-alkenyl group, a $C_3-C_4$-alkynyl group, a $C_1-C_3$-alkoxy($C_1-C_4$)-alkyl group, a $C_1-C_3$-mono- or polyhaloalkyl group, a $C_3-C_8$-cycloalkyl group, a benzyl group or a phenyl group; and $R^2$ and $R^3$ are the same or different and each is a hydrogen atom, a $C_1-C_6$-alkyl group, a $C_3-C_4$-alkenyl group, a $C_3-C_4$-alkynyl group, a $C_3-C_8$-cycloalkyl group, a benzyl group in which at most two of hydrogen atoms at the $\alpha$-position thereof may be substituted by methyl group, a $C_2-C_3$-hydroxyalkyl group or a phenyl group in which at most three of hydrogen atoms thereof may be substituted by the same or different $C_1-C_2$-alkyl group or halogen atom;

Y is a $C_1-C_4$-mono- or polyhaloalkyl group;

$A^1$ and $A^2$ are the same or different and each is a hydrogen atom, a fluorine atom, a chlorine atom, a bromine atom, a trihalomethyl group, a $C_1-C_2$-alkylthio group or a $C_1-C_2$-haloalkoxy group; and

W and Z are C-F and N-H respectively with non-bonding, or are taken together to form C-N, provided that X is $OR^1$ in case that W and Z are C-F and N-H respectively,

and an inert carrier or diluent.

Preferably used according to the present invention are compounds of formula (I) as defined in subclaims 2 to 9.

According to a further preferred embodiment of the present invention the plant male sterilant contains as the active ingredient a compound having the general formula (II)

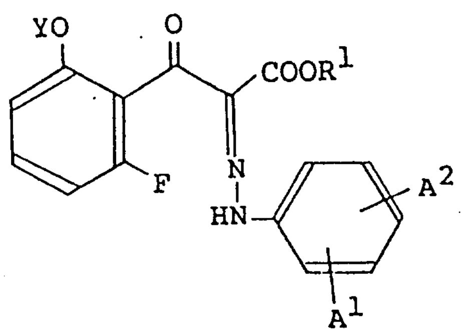

(II)

wherein

$R^1$ is a $C_1-C_9$-alkyl group, a $C_3-C_6$-alkenyl group, a $C_3-C_4$-alkynyl group, a $C_1-C_3$-alkoxy($C_1-C_4$)-alkyl group, a $C_1-C_3$-mono- or polyhaloalkyl group, a $C_3-C_8$-cycloalkyl group, a benzyl group or a phenyl group;

Y is a $C_1-C_4$-mono- or polyhaloalkyl group; and

$A^1$ and $A^2$ are the same or different and each is a hydrogen atom, a fluorine atom, a chlorine atom, a bromine atom, a trihalomethyl group, a $C_1-C_2$-alkylthio group or a $C_1-C_2$-haloalkoxy group.

According to the present invention preferably used as an active ingredient are compounds of formula (II) as defined in subclaims 11 and 12.

A further subject of the present invention is a method for inducing male sterlility in a plant which comprises applying an effective amount of a compound having the general formula (I) or (II) as defined above and an inert carrier or diluent to the plant.

The plant preferably used is wheat or rice.

According to a further aspect the present invention relates to new hydrazone derivatives having the general formula (II):

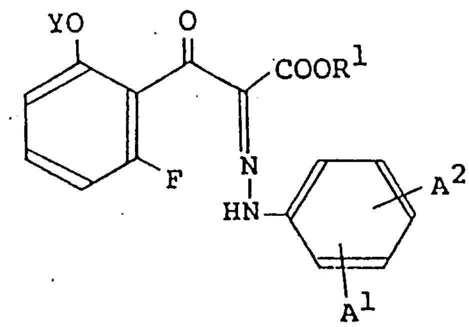

(II)

wherein

$R^1$ is a $C_1-C_9$-alkyl group, a $C_3-C_6$-alkenyl group, a $C_3-C_4$-alkynyl group, a $C_1-C_3$-alkoxy-$(C_1-C_4)$-alkyl group, a $C_1-C_3$-mono- or polyhaloalkyl group, a $C_3-C_8$-cycloalkyl group, a benzyl group or a phenyl group;

Y is a $C_1-C_4$ mono- or polyhaloalkyl group; and

$A^1$ and $A^2$ are the same or different and each is a hydrogen atom, a fluorine atom, a chlorine atom, a bromine atom, a trihalomethyl group, a $C_1-C_2$-alkylthio group or a $C_1-C_2$-haloalkoxy group.

Preferred hydrazone derivatives of formula (II) are the compounds defined in subclaims 16 and 17.

Hereinafter, the present invention is explained in detail.

Among the plant male sterilants of the present invention, those wherein W and Z are taken together to form C-N are preferred in efficiency. More preferred among them are those in which X is $-OH$, $-O^-M^+$ or $-OR^1$.

Further, particularly more preferred among them are those in which $A^1$ is a fluorine atom, a chlorine atom, a bromine atom, a trifluoromethyl group, a difluoromethoxy group or a trifluoromethoxy group and $A^2$ is a hydrogen atom or a fluorine atom.

Among them, the most preferred are those wherein Y is a $C_1-C_2$-polyfluoroalkyl group, e.g. a difluoromethyl group, a trifluoromethyl group, a 1,1,2,2-tetrafluoroethyl group or a 2,2,2-trifluoroethyl group.

The plant male sterilant of the present invention is used for various cultivated plants, for instance, grains such as rice, wheat, barley, oat, rye and corn, leguminous crops such as soybean, vegetables such as eggplant, tomato and cabbage or flower and ornamental plants such as morningglory, petunia and zinnia.

The plant male sterilant of the present invention can sufficiently induce male sterility in plant without causing any serious phytotoxicity on the plant. That is to say, when the plant male sterilant of the present invention is used, it can induce almost completely male sterility in plant without causing any undesirable side-effects on plant.

Further, as mentioned in the following Test Examples, since the plant male sterilant of the present invention has no harmful influence on a pistil, the hybrid seeds can be easily obtained by means of cross-pollination.

Processes for preparing the compounds used as an active ingredient in the plant male sterilant of the present invention are explained below.

Among the compounds, a compound in which W and Z are C-F and N-H respectively with non-bonding, i.e. a hydrazone derivative having the general formula (II), are obtained by reacting a benzoylacetate derivative having the formula (III):

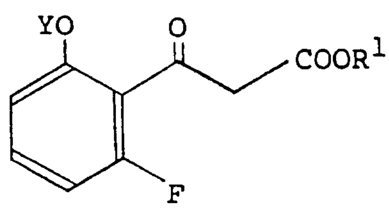

(III)

wherein $R^1$ and Y are as defined above, with a diazonium salt derivative having the formula (IV):

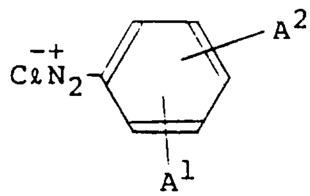

(IV)

wherein $A^1$ and $A^2$ are as defined above.

The reaction is usually carried out in a solvent at a temperature of 0 to 50 °C for a period of 10 minutes to 10 hours. The compound (IV) may be used in an amount of about 1 to about 1.5 equivalents to one equivalent of the compound (III).

Examples of the solvent are, for instance, ethers (e.g. diethyl ether, diisopropyl ether, dioxane, tetrahydrofuran and diethylene glycol dimethyl ether), alcohols (e.g. methanol, ethanol, isopropanol, t-butanol, octanol, cyclohexanol, methyl cellosolve, diethylene glycol and glycerin), tertiaryamines (e.g. pyridine, triethylamine, N,N-diethylaniline, tributylamine and N-methylmorpholine), acid amides (e.g. formamide, N,N-dimethylformamide, acetamide), and water. Their mixtures are also usable.

Inorganic bases such as sodium carbonate, potassium carbonate, sodium acetate and potassium acetate may be added to the reaction mixture in order that the reaction proceeds smoothly.

After completion of the reaction, the reaction mixture is subjected to ordinary post-treatments such as extraction with an organic solvent and concentration. If desired, any conventional purification procedures such as chromatography and recrystallization may be adopted.

Among the compounds (II), which are obtainable according to the above process, preferred are those in which $A^1$ is a fluorine atom, a chlorine atom, a bromine atom, a trifluoromethyl group, a difluoromethoxy group or a trifluoromethoxy group and $A^2$ is a hydrogen atom or a fluorine atom.

Further, particulary more preferred among them are those wherein Y is a $C_1-C_2$ polyfluoroalkyl group.

The compound (IV) is obtainable according to the ordinary methods described in, for instance, Organic Functional Group Preparations, S.R. Sandler and W. Karo, Academic Press, Chapter 15(1968).

The compounds, in which W and Z are taken together to form C-N, can be prepared as follows:

(1) A cinnoline-3-carboxylate derivative in which X on the formula (I) is $-OR^1$, i.e. having the formula (I-1) :

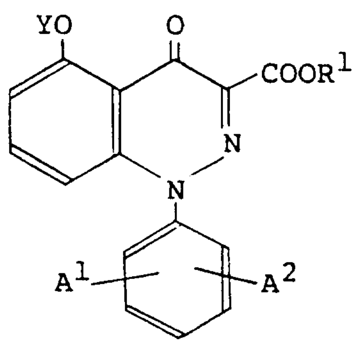

(I-1)

wherein $R^1$, Y, $A^1$ and $A^2$ are as defined above, is obtainable by subjecting a hydrazone derivative having the formula (V):

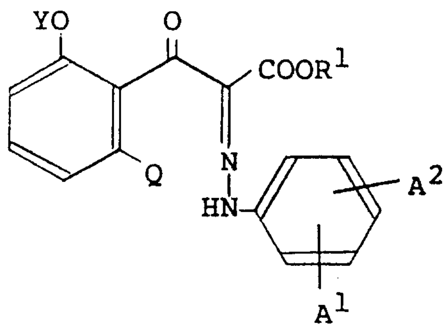

(V)

wherein $R^1$, Y, $A^1$ and $A^2$ are as defined above and Q is a halogen atom to ring closure with a dehydrohalogenating agent.

The reaction is carried out by using the dehydrohalogenating agent, in a solvent or without any solvent, at a temperature of 0 to 150 ° C for a period of 10 minutes to 20 hours. The dehydrohalogenating agent may be used in an amount of 1 to 10 equivalents to one equivalent of the compound (V).

In order to carry out the reaction more efficiently, quaternary ammonium salts or crown ethers may be added to the reaction mixture.

Examples of the solvent are, for instance, aliphatic hydrocarbons (e.g. hexane, heptane, ligroin, petroleum ether), aromatic hydrocarbons (e.g. benzene, toluene, xylene), halogenated hydrocarbons (e.g. chloroform, carbon tetrachloride, dichloroethane, chlorobenzene, dichlorobenzene), ethers (e.g. diethyl ether, diisopropyl ether, dioxane, tetrahydrofuran, diethylene glycol dimethyl ether), ketones (e.g. acetone, methyl ethyl ketone, methyl isobutyl ketone, isophorone, cyclohexanone), esters (e.g. ethyl formate, ethyl acetate, butyl acetate, diethyl carbonate), nitro compounds (e.g. nitroethane, nitrobenzene), nitriles (e.g. acetonitrile, isobutylonitrile), tertiary amines (e.g. pyridine, triethylamine, N,N-diethylaniline, tributylamine, N-methylmorphorine), acid amides (e.g. formamide, N,N-dimethylformamide, acetamide), sulfur compounds (e.g. dimethyl sufoxide, sulfolane), and water,. Their mixtures are also usable.

Examples of the dehydrohalogenating agent are, for instance, organic bases (e.g. pyridine, triethylamine, N,N-diethylaniline), inorganic bases (e.g. sodium hydroxide, potassium hydroxide, sodium carbonate, potassium carbonate, sodium hydride), and alkali metal alkoxide (e.g. sodium methoxide, sodium ethoxide).

As for the quaternary ammonium salts, there are, for instance, benzyl triethyl ammonium chloride and tetrabutyl ammonium chloride. Examples of the crown ether are, for instance, dibenzo-18-crown-6.

After completion of the reaction, the reaction mixture is subjected to ordinary post-treatments such as collection of crystals by means of addition of water, extraction with an organic solvent and concentration. If desired, purification procedures such as chromatography and recrystallization may be adopted to obtain the compound (I-1).

(2) A cinnoline-3-carboxylic acid derivative, in which X on the formula (I) is hydroxyl group, i.e. having the formula (I-2):

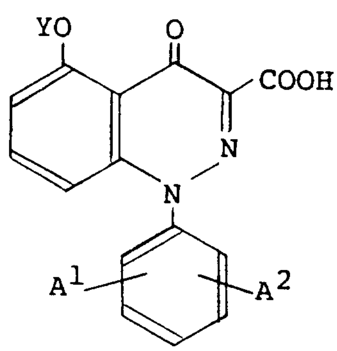

(I-2)

wherein Y, $A^1$ and $A^2$ are as defined above, can be prepared by subjecting the compound (I-1) to hydrolysis.

The reaction is usually carried out in either water or a mixture of water and other solvents such as alcohols (e.g. methanol, ethanol, isopropanol, diethylene glycol, glycerin), ketones (e.g. acetone), ethers (e.g. tetrahydrofuran, dioxane), nitriles (e.g. acetonitrile), acid amides (e.g. formamide, N,N-dimethylformamide) and sulfur compounds (e.g. dimethyl sulfoxide).

Usually, there is added acids or alkalis to the reaction mixture in an amount of about 1 to about 100 equivalents to one equivalent of the compound (I-1).

The reaction can be accomplished at a temperature of 20 to 100 ° C for a period of 30 minutes to 10 hours. Examples of the acids are, for instance, hydrochloric acid, sulfuric acid, and nitric acid.

Examples of the alkalis are, for instance, sodium hydroxide, and potassium hydroxide,.

In case of using the alkalis, after completion of the reaction, the reaction mixture is sujected to the neutralization with acids such as hydrochloric acid, sulfuric acid, formic acid and acetic acid. As for post-treatment of the reaction mixture, if there are precipitated crystals, they are collected by filtration, otherwise the compound (I-2) is obtainable by subjecting the reaction mixture to purification procedures such as extraction with an organic solvent and concentration.

(3) A cinnoline-3-carboxylic acid derivative, in which X on the formula (I) is $-O^{-}M'^{+}$, i.e. having the formula (I-3) :

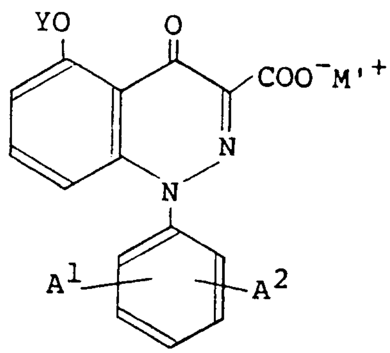

(I-3)

wherein Y, $A^1$ and $A^2$ are as defined above and $M'^{+}$ is an alkali metal cation or an alkaline earth metal cation, can be prepared by reacting the compound (I-2) with a metal hydroxide having the formula (VI):

$M'^{+}OH^{-}$ (VI)

wherein $M'^{+}$ is as defined above.

Examples of the metal hydroxide are, for instance, lithium hydroxide, sodium hydroxide, potassium hydroxide, and calcium hydroxide,.

The reaction is usually carried out in water and the metal hydroxide may be employed in an amount of about 0.7 to about 1 equivalent to one equivalent of the compound (I-2).

The reaction can be accomplished at a temperature of 0 to 50 ° C for a period of 5 minutes to 5 hours. After completion of the reaction, if desired, a water layer is washed with an organic solvent and the compound (I-3) is obtainable by concentrating the water layer.

(4) A cinnoline-3-carboxylic acid amine salt derivative, in which X on the formula (I) is

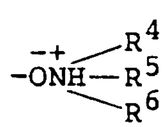 ,

i.e. having the formula (I-4):

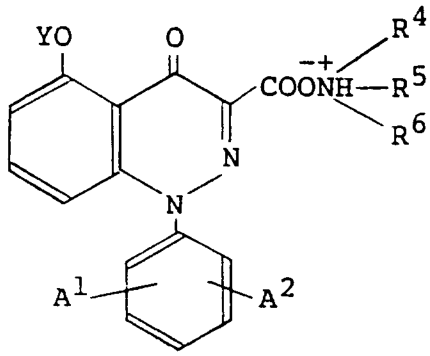

(I-4)

wherein $R^4$, $R^5$, $R^6$, Y, $A^1$ and $A^2$ are as defined above, can be prepared by reacting the compound (I-2) with an amine having the formula (VII) :

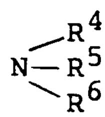

(VII)

wherein $R^4$, $R^5$ and $R^6$ are as defined above.

The reaction is usually carried out in a solvent or without any solvent at a temparature of 0 to 100°C for a period of 5 minutes to 8 hours. The amine(VII) may be used in an amount of 1 to 10 equivalents to one equivalent of the compound (I-2).

Examples of the solvent are, for instance, aromatic hydrocarbons (e.g. benzene, toluene, xylene), halogenated hydrocarbons (e.g. chloroform, carbon tetrachloride, dichloroethane, chlorobenzene, dichlorobenzene), ethers (e.g. diethyl ether, diisopropyl ether, dioxane, tetrahydrofuran, diethylene glycol dimethyl ether), alcohols (e.g. methanol, ethanol, isopropanol, t-butanol, octanol, cyclohexanol, methyl cellosolve, diethylene glycol, glycerin), esters (e.g. ethyl formate, ethyl acetate, butyl acetate, diethyl carbonate), nitro compounds (e.g. nitro ethane, nitro benzene), nitriles (e.g. acetonitrile, isobutylonitrile), and water,. Their mixtures are also usable.

After completion of the reaction, the reaction mixture is subjected to ordinary post-treatments such as concentration and, if necessary, to purification procedures such as recrystallization to give the compound (I-4).

(5) A cinnoline-3-carboxylic acid amide derivative, in which X on the formula (I) is

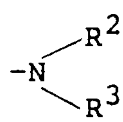

,

i.e. having the formula (I-5) :

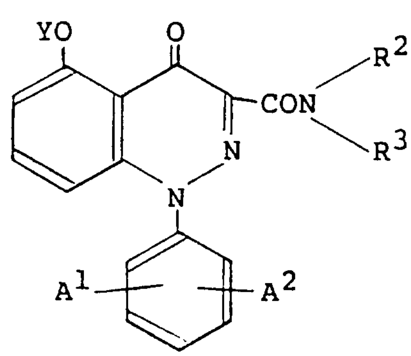

(I-5)

wherein $R^2$, $R^3$, Y, $A^1$ and $A^2$ are as defined above, can be prepared by reacting a cinnoline-3-carboxylic acid halide having the formula (VIII):

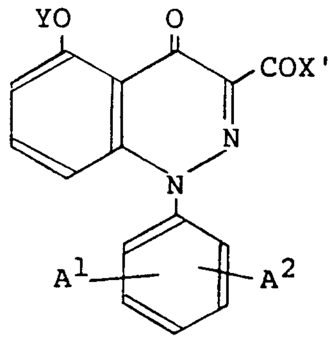

(VIII)

wherein Y, $A^1$ and $A^2$ are as defined above and X' is a halogen atom, with an amine having the formula (IX):

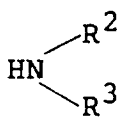

(IX)

wherein $R^2$ and $R^3$ are as defined above.

The reaction is usually carried out in a solvent or without any solvent, in the presense of a dehydrohalogenating agent at a temperature of 0 to 50°C for a period of 10 minutes to 3 hours. The compound (IX) and the dehydrohalogenating agent may be used in an amount of 1 to 5 equivalents to and 1 to 2 equivalents to one equivalent of the compound (VIII) respectively.

Examples of the solvent are, for instance, aliphatic hydrocarbons (e.g. hexane, heptane, ligroin, petroleum ether), aromatic hydrocarbons (e.g. benzene, toluene, xylene), halogenated hydrocarbons (e.g. chloroform, carbon tetrachloride, dichloroethane, chlorobenzene, dichlorobenzene), ethers (e.g. diethyl ether, diisopropyl ether, dioxane, tetrahydrofuran, diethylene glycol dimethyl ether), esters (e.g. ethyl formate, ethyl acetate, butyl acetate, diethyl carbonate), nitro compounds (e.g. nitroethane, nitrobenzene), nitriles (e.g. acetonitrile, isobutylonitrile), tertiary amines (e.g. pyridine, triethylamine, N,N-diethylaniline, tributylamine, N-methylmorphorine), acid amides (e.g. formamide, N,N-dimethylformamide, acetamide), sulfur compounds (e.g. dimethyl sulfoxide, sulfolane), and water,. The mixtures of them are also usable.

Examples of the dehydrohalogenating agent are organic bases such as pyridine, triethylamine, N,N-diethylaniline.

After completion of the reaction, the reaction mixture is subjected to ordinary post-treatments such as extraction with an organic solvent and concentration to obtain the compound (I-5). If necessary, purification procedures such as chromatography and recrystallization may be adopted.

On the other hand, the compound (VIII) can be easily obtained by subjecting the compound (I-2) to ordinary acid halogenation.

Typical examples of the compound contained in the plant male sterilant of the present invention, which can be prepared through the above procedures, are shown in Table 1 and Table 2.

Table 1

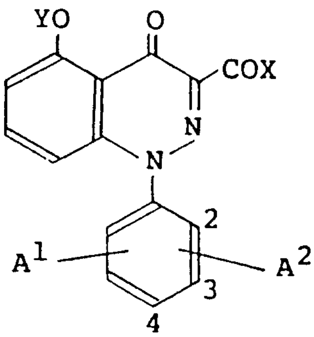

| $A^1$ | $A^2$ | X | Y |
|---|---|---|---|
| H | H | OH | $CHF_2$ |
| " | " | OK | " |
| " | " | $OC_2H_5$ | " |
| 2-Cl | " | " | " |
| 4-Cl | " | " | " |
| " | " | OH | " |
| " | " | OK | " |
| " | " | $ONH_4$ | " |
| 4-Cl | " | $N(CH_3)_2$ | " |
| 2-F | 4-Cl | $OC_4H_9$-n | " |
| " | " | $OC_2H_5$ | " |
| 3-Cl | 4-F | " | " |
| 2-Cl | 4-Cl | " | " |
| 4-Br | H | OH | " |
| " | " | 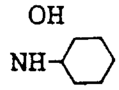 | " |
| 2-F | 4-Br | $OC_3H_7$-i | " |
| 4-$CF_3$ | H | OH | " |
| " | " | ONa | " |

- continued -

- continued -

| $A^1$ | $A^2$ | X | Y |
|---|---|---|---|
| 4-$CF_3$ | H | $OCH_3$ | $CHF_2$ |
| 4-$SCH_3$ | " | OH | " |
| " | " | OK | " |
| " | " | $OC_2H_5$ | " |
| 4-$OCHF_2$ | " | $OCH_3$ | " |
| 2-F | 4-$OCHF_2$ | OK | " |
| 3-$OCF_3$ | H | $OC_2H_5$ | " |
| 4-$OCF_3$ | " | " | " |
| " | " | OH | " |
| " | " | ONa | " |
| " | " | $O(\frac{1}{2}Ca)$ | " |
| " | " | $ONH(C_2H_5)_3$ | " |
| 4-$OCF_2Br$ | " | $OC_2H_5$ | " |
| 4-$OCF_2CHCl_2$ | " | " | " |
| H | " | OH | $CF_3$ |
| " | " | ONa | " |
| " | " | $OC_2H_5$ | " |
| 4-F | " | " | " |
| 4-Cl | " | OH | " |
| " | " | OK | " |
| " | " | $OC_2H_5$ | " |
| " | " | 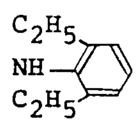 | " |
| 3-F | 4-Cl | OH | " |
| " | " | OK | " |
| " | " | $OCH_3$ | " |
| " | " | $OC_4H_9$-n | " |
| 2-Cl | " | $OC_2H_5$ | " |
| 4-Br | H | $OCH_3$ | " |
| 2-F | 4-Br | $OC_2H_5$ | " |
| 3-$CF_3$ | H | $OCH_3$ | " |

- continued -

- continued -

| $A^1$ | $A^2$ | X | Y |
|---|---|---|---|
| 4-$CF_3$ | H | $OC_3H_7$-i | $CF_3$ |
| 4-$SCH_3$ | " | OH | " |
| " | " | ONa | " |
| " | " | $OC_2H_5$ | " |
| 4-$OCHF_2$ | " | " | " |
| 2-$OCHF_2$ | 4-F | $OCH_3$ | " |
| 4-$OCF_3$ | H | OH | " |
| " | " | OK | " |
| " | " | $OC_2H_5$ | " |
| 4-$OCF_2Br$ | " | OK | " |
| H | " | $OC_2H_5$ | $CF_2Br$ |
| 4-Cℓ | " | OK | " |
| 2-F | 4-Cℓ | $OC_3H_7$-i | " |
| 4-$SCH_3$ | H | $OC_3H_7$-n | " |
| 4-$OCHF_2$ | " | OH | " |
| 4-$OCF_3$ | " | $OC_2H_5$ | " |
| H | " | OH | $CF_2CF_2H$ |
| " | " | ONa | " |
| " | " | $OC_2H_5$ | " |
| 2-Cℓ | " | $OCH_3$ | " |
| 4-Cℓ | " | $OC_2H_5$ | " |
| 2-F | 4-Cℓ | $OCH_3$ | " |
| 3-F | " | $OC_2H_5$ | " |
| 3-Cℓ | 4-F | OK | " |
| 4-Br | H | OH | " |
| 4-$CF_3$ | " | " | " |
| 4-$SCH_3$ | " | OK | " |
| " | " | $OCH_3$ | " |
| 4-$OCF_3$ | " | $OC_2H_5$ | " |
| 4-$CF_2Br$ | " | OH | " |
| 4-F | " | $OC_2H_5$ | $CH_2CF_3$ |
| 4-Cℓ | " | " | " |
| 4-Br | " | OH | " |

- continued -

- continued -

| $A^1$ | $A^2$ | X | Y |
|---|---|---|---|
| 4-$CF_3$ | H | OK | $CH_2CF_3$ |
| 4-$SCH_3$ | " | OH | " |
| 2-F | 4-$OCHF_2$ | $OC_2H_5$ | " |
| 2-Cℓ | 4-F | $OC_3H_7$-n | $CF_2CHCℓ_2$ |
| 3-Cℓ | " | $OCH_3$ | " |
| 4-$SCH_3$ | H | $OC_2H_5$ | " |
| 4-$OCF_3$ | " | OK | " |
| H | " | $OC_2H_5$ | $CF_2CHFCℓ$ |
| 4-F | " | " | " |
| " | " | OH | " |
| " | " | OK | " |
| 4-Br | " | $OCH_3$ | " |
| 2-F | 4-Br | OH | " |
| 4-$OCF_3$ | H | $OC_2H_5$ | " |
| 4-Cℓ | " | OK | CFCℓCHFCℓ |
| 3-Cℓ | 4-Cℓ | $OC_2H_5$ | " |
| 4-$CF_3$ | H | $OC_3H_7$-n | " |
| 4-$SCH_3$ | " | OH | " |
| 4-$OCHF_2$ | " | " | " |
| H | " | " | $CF_2CHFCF_3$ |
| 4-F | " | $OC_2H_5$ | " |
| 4-Cℓ | " | OK | " |
| 2-F | 4-Cℓ | $OCH_3$ | " |
| 4-$CF_3$ | H | $OC_2H_5$ | " |
| 4-$SC_2H_5$ | " | OH | " |
| 4-$OCF_3$ | " | ONa | " |

Table 2

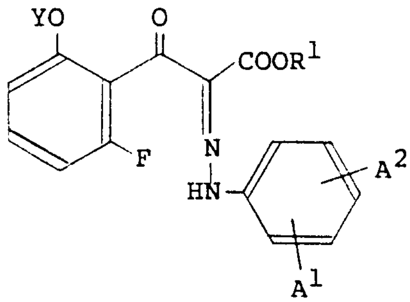

| $A^1$ | $A^2$ | $R^1$ | Y |
|---|---|---|---|
| H | H | $C_2H_5$ | $CHF_2$ |
| 2-Cl | " | " | " |
| 4-Cl | " | " | " |
| 2-F | 4-Cl | " | " |
| 2-Cl | 4-F | " | " |
| 2-F | 4-Br | " | " |
| 4-$CF_3$ | H | " | " |
| 4-$SCH_3$ | " | $CH_3$ | " |
| 4-$OCHF_2$ | " | $C_2H_5$ | " |
| 4-$OCF_3$ | " | " | " |
| 4-$OCF_2Br$ | " | " | " |
| 4-F | " | " | $CF_3$ |
| 4-Cl | " | " | " |
| 2-Cl | " | " | " |
| 3-F | 4-Cl | $C_4H_9$-n | " |
| 4-Br | H | $CH_3$ | " |
| 2-F | 4-Br | $C_2H_5$ | " |
| 3-$CF_3$ | H | $CH_3$ | " |
| 4-$CF_3$ | " | $C_3H_7$-i | " |
| 4-$SCH_3$ | " | $C_2H_5$ | " |
| 4-$OCHF_2$ | " | " | " |
| 4-$OCF_3$ | " | " | " |
| 4-$OCF_2Br$ | " | $CH_3$ | " |
| H | " | $C_2H_5$ | $CF_2Br$ |

- continued -

- continued -

| $A^1$ | $A^2$ | $R^1$ | Y |
|---|---|---|---|
| 4-Cl | H | $CH_3$ | $CF_2Br$ |
| 2-F | 4-Cl | $C_3H_7$-i | " |
| 4-$SCH_3$ | H | $C_3H_7$-n | " |
| 4-$OCHF_2$ | " | " | " |
| 4-$OCF_3$ | " | $C_2H_5$ | " |
| H | " | " | $CF_2CF_2H$ |
| 2-Cl | " | $CH_3$ | " |
| 4-Cl | " | $C_2H_5$ | " |
| 2-F | 4-Cl | $CH_3$ | " |
| 3-F | " | $C_2H_5$ | " |
| 3-Cl | 4-F | " | " |
| 4-Br | H | " | " |
| 4-$SCH_3$ | " | $CH_3$ | " |
| 4-$OCF_3$ | " | $C_2H_5$ | " |
| 4-$OCF_2Br$ | " | " | " |
| 4-F | " | " | $CH_2CF_3$ |
| 4-Cl | " | " | " |
| 4-Br | " | $CH_3$ | " |
| 4-$CF_3$ | " | " | " |
| 4-$SCH_3$ | " | $C_2H_5$ | " |
| 2-Cl | 4-F | $C_3H_7$-n | " |
| 3-Cl | " | $CH_3$ | " |
| 4-$SCH_3$ | H | $C_2H_5$ | " |
| 2-F | 4-$OCHF_2$ | " | " |
| 4-$OCF_3$ | H | " | " |
| " | " | " | $CF_2CHFCl$ |
| 4-F | " | " | " |
| 4-Br | " | $CH_3$ | " |
| 2-F | 4-Br | $C_2H_5$ | " |
| 4-$OCF_3$ | H | " | " |
| 4-Cl | " | $CH_3$ | CFClCHFCl |
| 3-Cl | 4-Cl | $C_2H_5$ | " |

- continued -

- continued -

| $A^1$ | $A^2$ | $R^1$ | Y |
|---|---|---|---|
| 4-$CF_3$ | H | $C_3H_7$-n | CFCℓCHFCℓ |
| 4-$SCH_3$ | " | $C_2H_5$ | " |
| 4-$OCHF_2$ | " | $C_4H_9$-n | " |
| H | " | $CH_3$ | $CF_2CHFCF_3$ |
| 4-F | " | $C_2H_5$ | " |
| 4-Cℓ | " | " | " |
| 2-F | 4-Cℓ | $CH_3$ | " |
| 4-$CF_3$ | H | $C_2H_5$ | " |
| 4-$SC_2H_5$ | " | $C_4H_9$-sec | " |
| 4-$OCF_3$ | " | $C_2H_5$ | " |

The compound having the formula (V) can be prepared in accordance with the process for preparing the compound having the formula (II).

Further, a benzoylacetate derivative having the formula (X):

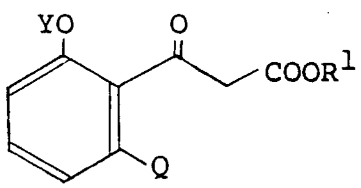

(X)

in which $R^1$, Y and Q are as defined above, which is a starting material of the compound having the formula (V), can be prepared in accordance with the following steps.

[Step 1]

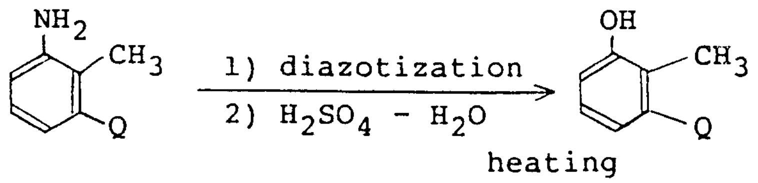


[Step 2]

Case (i) Y : $CF_3$

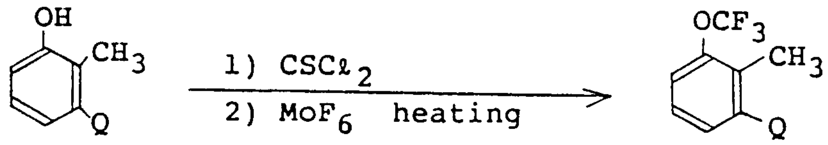


Case (ii) Y : $CH_2CF_3$

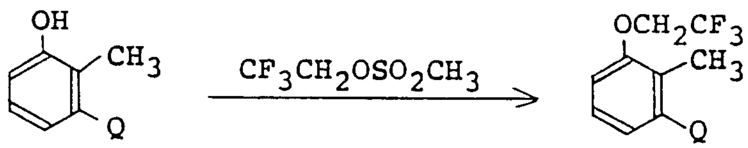


Case (iii) Y : a $C_1-C_4$ - mono - or polyhaloalkyl group except $-CF_3$ and $-CH_2CF_3$

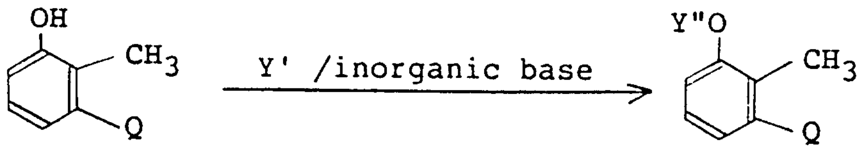

[Step 3]

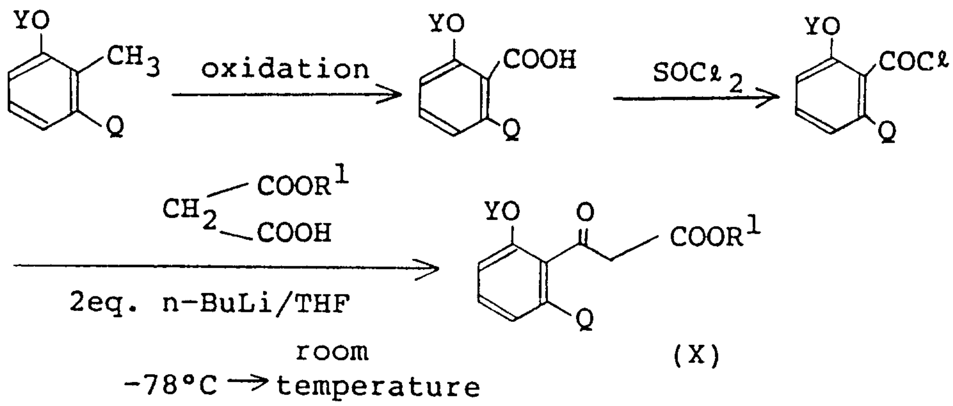


(X)

in which $R^1$, Q and Y are as defined above and Y' is a polyhalogenated hydrocarbon, which is capable of being added to a phenol, and Y'' is a $C_1-C_4$ mono-or polyhaloalkyl group except $-CF_3$ and $-CH_2CF_3$.

The present invention is more specifically described and explained by means of the following Examples, wherein the compound number of the active ingredient corresponds to the one in Table 3 and Table 4.

Example 1

[Preparation of the compound No. 55]

To 237 mg of 2-fluoro-4-chloroaniline were added 3 mℓ of water and 1 mℓ of concentrated hydrochloric acid to prepare a solution of hydrochloric acid salt. And thereto, a solution of 124 mg of sodium nitrite in 2 mℓ of water was added dropwise over about 5 minutes while cooling with ice to form a diazonium salt.

The obtained solution was added dropwise to a solution of 500 mg (purity: 90 %) of ethyl 2-fluoro-6-difluoromethoxybenzoylacetate in a mixture of 8 mℓ of 70 % methanol and 0.8 mℓ of pyridine at 10 to 20 °C over about 10 minutes, followed by stirring the reaction mixture for 1 hour at room temperature.

After being added to 30 mℓ of ice-water containing 2 mℓ of a 1N hydrochloric acid, the resultant mixture was extracted twice with 30 mℓ of ethyl acetate. The extract was washed with 20 mℓ of water and 20 mℓ of saturated saline solution successively and was dried over anhydrous magnesium sulfate. The solid obtained by removing ethyl acetate under reduced pressure was washed with a mixed solvent of hexane and ethanol to give 500 mg of desired ethyl 2-[(2-fluoro-4-chlorophenyl)-1,1-diazanediyl]-(2-fluoro-6-difluoromethoxybenzoyl)acetate (yield: 70.9 %, m.p.: 78-80 °C).

Example 2

[Preparation of the compound No. 61]

To 289 mg of 4-trifluoromethoxyaniline were added 3 mℓ of water and 1 mℓ of concentrated hydrochloric acid. And thereto, a solution of 124 mg of sodium nitrite in 2 mℓ of water was added dropwise while cooling with ice over about 5 minutes to prepare a solution of diazonium salt.

The obtained solution was added dropwise to a solution of 500 mg of ethyl 2-fluoro-6-difluoromethoxybenzoylacetate (purity: 90 %) in a mixture of 8 mℓ of 70 % methanol and 0.8 mℓ of pyridine at 10 to 20 °C over about 10 minutes, followed by stirring the resultant solution for 1 hour at room temperature.

After being added to 30 mℓ of ice-water added 2 mℓ of a 1N hydrochloric acid, the reaction mixture was extracted twice with 30 mℓ of ethyl acetate. The extract was washed with 20 mℓ of water and 20 mℓ of saturated saline solution successively and was dried over anhydrous magnesium sulfate. The solid obtained by removing ethyl acetate under reduced pressure was washed with a mixed solvent of hexane and ethanol to give 530 mg of desired ethyl 2-[(4-trifluoromethoxyphenyl)-1,1-diazandiyl]-(2-fluoro-6-

difluoromethoxybenzoyl)acetate (yield: 70 %, m.p.: 80.5°C)

Example 3

[Preparation of the compound No. 33]

There were added 530 mg of ethyl 2-[(4-trifluoromethoxyphenyl)-1,1-diazanediyl]-(2-fluoro-6-difluoromethoxybenzoyl)acetate and 157 mg of potassium carbonate to 10 mℓ of N,N-dimethylformamide, and the resultant mixture was heated for 1 hour at 100°C.

After cooling the reaction mixture to room temperature, the mixture was poured into about 50 mℓ of ice-water. After being allowed to stand overnight, precipitated crystals were collected by filtration.

The crystals were washed twice with 5 mℓ of water and then dried under reduced pressure to give 478 mg of desired ethyl 1-(4-trifluoromethoxyphenyl)-1,4-dihydro-4-oxo-5-difluoromethoxycinnoline-3-carboxylate (yield: 94.3 %, m.p.: 145°C).

Example 4

[Preparation of the compound No. 15]

There were added 460 mg of ethyl 2-[(2-fluoro-4-chlorophenyl)-1,1-diazanediyl]-(2-fluoro-6-difluoromethoxybenzoyl)acetate and 137 mg of potassium carbonate to 10 mℓ of N,N-dimethylformamide, and the reaction mixture was heated for 1 hour at 100°C.

After cooling the mixture to room temperature, the mixture was poured into about 50 mℓ of ice-water. After being allowed to stand overnight, the precipitated crystals were collected by filtration, washed twice with 5 mℓ of water and then dried under reduced pressure to give 410 mg of desired ethyl 1-(2-fluoro-4-chlorophenyl)-1,4-dihydro-4-oxo-5-difluoromethoxycinnoline-3-carboxylate (yield: 93.8 %, m.p.: 140-142°C).

Example 5

[Preparation of the compound No. 30]

There were added 200 mg of ethyl 1-(4-trifluoromethoxyphenyl)-1,4-dihydro-4-oxo-5-difluoromethoxycinnoline-3-carboxylate and 0.93 mℓ of a 1N aqueous solution of sodium hydroxide to a mixture of 5.5 mℓ of ethanol and 1.5 mℓ of water and then the resultant mixture was stirred for 3 hours at 70 to 80°C.

After cooling the reaction mixture to room temperature, the mixture was diluted with 50 mℓ of water and washed with 20 mℓ of ethyl acetate. Crystals were precipitated after adjusting the pH of the water layer to pH 2 with concentrated hydrochloric acid.

The crystals were collected by filtration, washed twice with 5 mℓ of water, and dried under reduced pressure to give 120 mg of desired 1-(4-trifluoromethoxyphenyl)-1,4-dihydro-4-oxo-5-difluoromethoxycinnoline-3-carboxylic acid (yield: 64.2 %, m.p. : 236.5°C).

Example 6

[Preparation of the compound No. 31]

There were added 120 mg of 1-(4-trifluoromethoxyphenyl)-1,4-dihydro-4-oxo-5-difluoromethoxycinnoline-3-carboxylic acid and 0.265 mℓof a 1N aqueous solution of sodium hydroxide to 5 mℓ of water and then the resultant mixture was stirred for 1 hour at room temperature. The reaction mixture was washed with 10 mℓ of ethyl acetate.

After removing the water, the obtained crystals were dried to give 91 mg of desired sodium 1-(4-trifluoromethoxyphenyl)-1,4-dihydro-4-oxo-5-difluoromethoxycinnoline-3-carboxylate (yield: 78.4 %, m.p. : 165 - 169°C).

Some examples of the present invention prepared in the same manner as the above are shown in Table 3 and Table 4.

Table 3

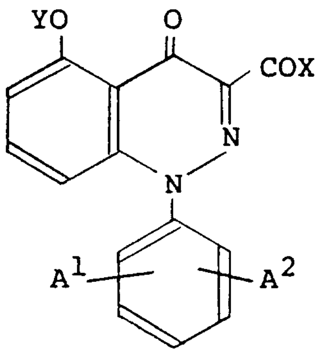

| Compound No. | $A^1$ | $A^2$ | X | Y | Melting point (°C ) |
|---|---|---|---|---|---|
| 1 | H | H | OH | $CHF_2$ | 261-263 |
| 2 | " | " | OK | " | 277-279 |
| 3 | " | " | $OC_2H_5$ | " | 159-161 |
| 4 | 4-F | " | ONa | " | 248-255 |
| 5 | " | " | $OC_2H_5$ | " | 185.5 |
| 6 | 2-Cl | " | " | " | 191.7 |
| 7 | 4-Cl | " | OH | " | 256.8 |
| 8 | " | " | OK | " | 270-275 |
| 9 | " | " | $OC_2H_5$ | " | 198.3 |

- continued -

- continued -

| Compound No. | $A^1$ | $A^2$ | X | Y | Melting point (°C ) |
|---|---|---|---|---|---|
| 10 | 4-Br | H | OH | $CHF_2$ | 260-263 |
| 11 | " | " | ONa | " | 211-215 |
| 12 | " | " | $OC_2H_5$ | " | 192-194 |
| 13 | 2-F | 4-Cl | OH | " | 206.5 |
| 14 | " | " | ONa | " | 240-246 |
| 15 | " | " | $OC_2H_5$ | " | 140-142 |
| 16 | " | 4-Br | OH | " | 208-210 |
| 17 | " | " | $OC_2H_5$ | " | 137-140 |
| 18 | 2-Cl | 4-F | " | " | 142-146 |
| 19 | " | 4-Cl | " | " | 176 |
| 20 | 3-Cl | 4-F | OH | " | 266-268 |
| 21 | " | " | ONa | " | 260-265 |
| 22 | " | " | $OC_2H_5$ | " | 138-144 |
| 23 | $4-CF_3$ | H | OH | " | 260-263 |
| 24 | " | " | ONa | " | 240-250 |
| 25 | " | " | OK | " | >300 |
| 26 | " | " | $OC_2H_5$ | " | 192.8 |
| 27 | $4-OCHF_2$ | " | OH | " | 237-241 |
| 28 | " | " | ONa | " | 183-187 |
| 29 | " | " | $OC_2H_5$ | " | 141.7 |
| 30 | $4-OCF_3$ | " | OH | " | 236.5 |
| 31 | " | " | ONa | " | 165-169 |
| 32 | " | " | OK | " | 267-270 |
| 33 | " | " | $OC_2H_5$ | " | 145 |
| 34 | $4-SCH_3$ | " | OH | " | 253-256 |
| 35 | " | " | $OC_2H_5$ | " | 152-156 |
| 36 | $4-OCF_2CHF_2$ | " | " | " | 162.1 |
| 37 | 4-Cl | " | OH | $CF_3$ | 264-268 |
| 38 | " | " | OK | " | 270-274 |
| 39 | " | " | $OC_2H_5$ | " | 207-209 |
| 40 | $4-OCF_3$ | " | OH | " | 269-270 |

- continued -

- continued -

| Compound No. | $A^1$ | $A^2$ | X | Y | Melting point (°C ) |
|---|---|---|---|---|---|
| 41 | 4-$OCF_3$ | H | OK | $CF_3$ | 205-210 |
| 42 | " | " | $OC_2H_5$ | " | 145-148 |
| 43 | 4-Cℓ | " | " | $CH_2CF_3$ | 172.9 |
| 44 | 4-Br | " | " | " | 176.6 |
| 45 | 4-Cℓ | " | OH | $CF_2CHF_2$ | 197-200 |
| 46 | " | " | OK | " | 265-272 |
| 47 | " | " | $OC_2H_5$ | " | 127-130 |
| 48 | 4-$OCF_3$ | " | OH | " | 254-261 |
| 49 | " | " | OK | " | 290-300 |
| 50 | " | " | $OC_2H_5$ | " | 147-149 |

Table 4

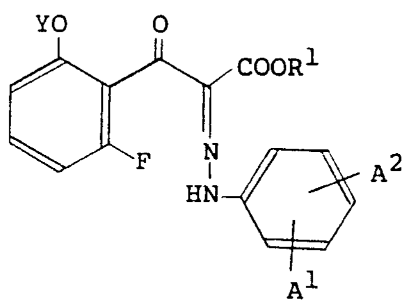

| Compound No. | $A^1$ | $A^2$ | $R^1$ | Y | Physical properties |
|---|---|---|---|---|---|
| 51 | 4-F | H | $C_2H_5$ | $CHF_2$ | m.p. 94.5-96.5°C |
| 52 | 2-Cℓ | " | " | " | m.p. 70.6°C |
| 53 | 4-Cℓ | " | " | " | $n_D^{23}$ 1.5890 |
| 54 | 4-Br | " | " | " | m.p. 108.7°C |
| 55 | 2-F | 4-Cℓ | " | " | m.p. 78-80°C |
| 56 | 3-Cℓ | 4-F | " | " | m.p. 55-60°C |

- continued -

- continued -

| Compound No. | $A^1$ | $A^2$ | $R^1$ | Y | Physical properties |
|---|---|---|---|---|---|
| 57 | 2-Cl | 4-Cl | $C_2H_5$ | $CHF_2$ | m.p. 76.2°C |
| 58 | 4-$CF_3$ | H | " | " | m.p. 77.6°C |
| 59 | 4-$OCHF_2$ | " | " | " | m.p. 78.3°C |
| 60 | 2-$OCHF_2$ | 4-F | " | " | $n_D^{22}$ 1.4531 |
| 61 | 4-$OCF_3$ | H | " | " | m.p. 80.5°C |
| 62 | 4-$SCH_3$ | " | " | " | m.p. 88-92°C |
| 63 | 4-$OCF_2CHF_2$ | " | " | " | $n_D^{18}$ 1.4719 |
| 64 | 4-Cl | " | " | $CF_3$ | m.p. 95-98°C |
| 65 | 4-$OCF_3$ | " | " | " | $n_D^{22}$ 1.5192 |
| 66 | 4-Cl | " | " | $CH_2CF_3$ | $n_D^{23}$ 1.5690 |
| 67 | 4-Br | " | " | " | $n_D^{22}$ 1.4813 |
| 68 | 4-Cl | " | " | $CF_2CHF_2$ | $n_D^{24}$ 1.4100 |
| 69 | 4-$OCF_3$ | " | " | " | $n_D^{24}$ 1.5270 |

Hereinafter, the method of the present invention for inducing male sterility in a plant is explained.

On the practical usage of the compounds as described above as an active ingredient of the plant male sterilant of the present invention, they can be applied in conventional preparation forms such as an emulsifiable concentrate, a wettable powder, a flowable, a granule and a liquid formulation in combination with a conventional solid carrier, liquid carrier, surface active agent or an auxiliary substance for formulation.

The content of the compounds of the present invention as the active ingredient in such preparations is within a range of 1 to 80 % by weight, preferably 2 to 70 % by weight.

Examples of the solid carrier, for instance, are fine powders or granules of kaolin clay, attapulgite clay, bentonite, terra alba, pyrophyllite, talc, diatomaceous earth, calcite, walnut powders, urea, ammonium sulfate and synthetic hydrous silicate.

As the liquid carrier, there may be exemplified aromatic hydrocarbons (e.g. xylene, methylnaphthalene), alcohols (e.g. isopropanol, ethylene glycol, cellosolve), ketones (e.g. acetone, cyclohexanone, isophorone), vegetable oils (e.g. soybean oil, cotton seed oil), dimethylsulfoxide, N,N-dimethylformamide, acetonitrile, and water.

Examples of the surface active agent used for emulsification, dispersion or spreading are, for instance, anionic type agents (e.g. alkylsulfates, alkylsulfonates, alkylarylsulfonates, dialkylsulfosuccinates, polyoxyethylenealkylaryl ether phosphates), and non-ionic type agents (e.g. polyoxyethylene alkyl ethers, polyoxyethylene alkylaryl ethers, polyoxyethylene polyoxypropylene block copolymer, sorbitan fatty acid esters, polyoxyethylene sorbitan fatty acid esters).

Examples of the auxiliary substance for formulation include ligninsulfonates, sodium alginate, polyvinyl alcohol, gum arabic, CMC (carboxymethyl cellulose), and PAP (isopropyl acid phosphate).

The compounds according to the present invention are usually formulated and applied to plant by foliar treatment, soil treatment or application on the surface of the water during the period of just before the reproductive growth to the flowering.

As for the application on the surface of the water, it is necessary to partition a male plant and a female plant, which are planted adjoining each other, so that the male sterilant is not absorbed by the male plant.

As for the foliar treatment and soil treatment, it is also necessary to keep the male sterilant off the male plant.

In case of using the compounds as an active ingredient of the plant male sterilant, the dosage rate thereof varies depending on weather conditions, formulation used, application timing, application method, soil involved, species or varieties of the plants treated, etc. Generally, however, the dosage rate is from 0.05 to 10,000 grams, preferably from 0.1 to 5,000 grams, of the active ingredient per ha.

The plant male sterilant of the present invention formulated in the form of an emulsifiable concentrate, a wettable powder, a flowable or a liquid formulation is ordinarily employed by diluting it with water at a volume of 1 to 10 liters per are, if necessary, with addition of auxiliary substances such as a spreading agent.

On the other hand, the plant male sterilant formulated in the form of granules may be normally applied without dilution.

Examples of the spreading agent include, in addition to the surface active agents as noted above, polyoxyethylene resin acid (ester), ligninsulfonate, abietic acid salt, dinaphthylmethanedisulfonate, and paraffin.

Further, the compounds of the present invention may be applied in combination with plant growth regulators, herbicides, insecticides, acaricides, nematocides, fungicides, fertilizers, and soil improvers.

Furthermore, the sterilant of the present invention can be treated several times with the same plant by changing the application timing.

In order to obtain a lot of hybrid seeds, it is applicable to employ a method as follows:

Two parent plants are planted alternately. A number of ridges or a width thereof of each parent plant varies depending on species or varieties of the plant treated, and environmental conditions. After applying the plant male sterilant of the present invention to female plant, the female plant, which is already male sterilized, are pollinated with pollens of male plant carried by wind, insects, and thereby the hybrid seeds can be obtained.

Practical embodiments of preparation of the plant male sterilant of the present invention are illustratively shown in the following Formulation Examples wherein all parts are by weight. The compound number of the active ingredient corresponds to the one in Table 3 and Table 4.

Formulation Example 1

Fifty parts of any one of Compound Nos. 3, 5, 9, 15, 22 and 66, 3 parts of calcium ligninsulfonate, 2 parts of sodium laurylsulfate and 45 parts of synthetic hydrous silicate are well mixed while being powdered to obtain a wettable powder.

Formulation Example 2

Ten parts of any one of Compound Nos. 3, 5, 9, 12, 15, 17, 18, 19, 22, 26, 29, 33, 35, 36, 39, 42, 43, 44, 47, 50, 53, 61 and 66, 14 parts of polyoxyethylenestyrylphenyl ether, 6 parts of calcium dodecylbenzenesulfonate and 70 parts of xylene are well mixed to obtain an emulsifiable concentrate.

Formulation Example 3

Two parts of any one of Compound Nos. 12, 17, 18, 19 and 53, 1 part of synthetic hydrous silicate, 2 parts of calcium ligninsulfonate, 30 parts of bentonite and 65 parts of kaolin clay are well mixed while being powdered. The mixture is then kneaded with water, granulated and dried to obtain granules.

Formulation Example 4

Twenty-five parts of any one of Compound Nos. 1, 7, 10, 22, 26, 27, 45, 51 and 53 is mixed with 3 parts of polyoxyethylene sorbitan monooleate, 3 parts of carboxymethyl cellulose and 69 parts of water and pulverized until the particle size of the mixture becomes less than 5 microns to obtain a flowable.

Formulation Example 5

Three parts of any one of Compound Nos. 2, 4, 8, 11, 14, 21, 24, 25, 28, 31, 32, 38, 41, 46 and 49, 1 part of polyoxyethylenestyrylphenyl ether and 96 parts of water are well mixed to obtain a liquid formulation.

The biological data of the compounds as the active ingredient in the plant male sterilant of the present invention are shown in the following Test Examples, wherein the compound number of the active ingredient corresponds to the one in Table 3 and Table 4.

Test Example 1

[Sterility test of wheat]

Plastic pots (volume: 200 ml) were filled with artificial soil mix and seeds of wheat (variety: NORIN No.61) were sowed therein and grown in a greenhouse under the conditions of a day length of 15 hours and a temperature of 27° C (day) and 20° C (night).

A designed amount of the test compounds formulated in an emulsifiable concentrate or a liquid formulation was diluted with water containing a spreading agent, and the dilution was sprayed over the foliage of the test plants by means of a small hand sprayer at a spray volume of 10 liters per are three times to the same pot, i.e. 2 – 5 days before, 9 – 12 days before and 16 – 19 days before the first heading time of the test plant.

After the heading and flowering, artificial pollination was carried out as to 2 heads per pot of the pots which appeared to be sterile, using pollens obtained from the heads of untreated plants.

After ripening, there were harvested 4 heads per pot of no artificial pollination and 2 heads per pot of artificial pollination and each of spikelets and seeds thereof was counted.

The test was carried out in one pot per treatment.

The sterility rate was calculated according to the following expression:

Sterility Rate (%) = (1 – B/A) x 100

A: the number of seeds per spikelet of an untreated plant

B: the number of seeds per spikelet of a treated plant (no – artificial pollination)

The results are shown in Table 5 by the sterility rates (%). It is shown that the test compounds induced the complete or nearly complete sterility in the test plants. Also, the female fertility in the test plants was observed by carrying out the artificial pollination.

Table 5

| Compound No. | Dosage (g/ha) | Sterility rate(%) |
|---|---|---|
| 1 | 7.8 | 100 |
| 2 | 7.8 | 100 |
| 3 | 7.8 | 100 |
| 4 | 7.8 | 100 |
| 5 | 2.0 | 100 |
| 6 | 7.8 | 100 |
| 7 | 0.5 | 100 |
| 8 | 31.3 | 100 |
| 9 | 7.8 | 92 |
| 11 | 2.0 | 100 |
| 12 | 125 | 100 |
| 14 | 7.8 | 100 |
| 15 | 7.8 | 100 |

- continued -

- continued -

| Compound No. | Dosage (g/ha) | Sterility rate(%) |
|---|---|---|
| 19 | 31.3 | 100 |
| 23 | 7.8 | 100 |
| 24 | 2.0 | 100 |
| 25 | 7.8 | 100 |
| 26 | 2.0 | 100 |
| 27 | 7.8 | 100 |
| 28 | 7.8 | 100 |
| 29 | 7.8 | 100 |
| 30 | 7.8 | 100 |
| 31 | 0.5 | 100 |
| 32 | 0.5 | 100 |
| 33 | 2.0 | 100 |
| 38 | 31.3 | 100 |
| 39 | 125 | 90 |
| 41 | 125 | 95 |
| 42 | 31.3 | 100 |
| 43 | 7.8 | 95.4 |
| 44 | 31.3 | 100 |
| 46 | 31.3 | 100 |
| 53 | 125 | 100 |
| 61 | 125 | 100 |
| 66 | 3000 | 100 |

Test Example 2

[Sterility test of wheat]

Wheat were grown according to the same method as in Test Example 1.

A designed amount of the test compounds formulated in an emulsifiable concentrate or a liquid formulation was diluted with water containing a spreading agent, and the dilution was sprayed over the foliage of the test plants by means of a small hand sprayer at a spray volume of 10 liters per are once to a pot at 9 – 15 days before the first heading time of the test plants.

After the heading, flowering and ripening of the test plants, there were harvested 4 heads per pot and spikelets and seeds thereof were counted.

The test was carried out in one pot per treatment.

The sterility rate was calculated according to the same method as in Test Example 1.

The results are shown in Table 6 by the sterility rates (%). It is shown that the test compounds induced the complete sterility in the test plants.

Table 6

| Compound No. | Dosage (g/ha) | Sterility rate (%) |
|---|---|---|
| 10 | 2.0 | 100 |
| 16 | 7.8 | 100 |
| 17 | 7.8 | 100 |
| 18 | 7.8 | 100 |
| 21 | 500 | 100 |
| 22 | 125 | 100 |
| 27 | 31.3 | 100 |
| 34 | 2000 | 100 |
| 35 | 2000 | 100 |

Test Example 3

[Selectivity test as to male sterilizing effect and phytotoxicity using wheat]

Wheat were grown according to the same method as in Test Example 1. A designed amount of the test compounds was sprayed over the foliage of the test plants according to the same methods as in Test Example 1.

After the heading, flowering and ripening, there were harvested 4 heads per pot and spikelets and seeds thereof were counted.

The sterility rate was calculated according to the same method as in Test Example 1 and rated with an index A, B or C, each of which is equivatent to 80 – 100 %, 50 – 79 % or not more than 50 % respectively.

The phytotoxicity to heads were observed visually and rated with an index A, B or C, in which "A" indicates the phytotoxicity is hardly noticeable, "B" indicates the phytotoxicity is acceptable (phytotoxic against glume but no phytotoxic against pistil) and "C" indicates the phytotoxicity is not acceptable (phytotoxic against pistil).

The test was carried out in one pot per treatment.

The results were shown in Table 7.

Table 7

| Compound No. | | Dosage (g/ha) 0.12 | 0.5 | 2.0 | 7.8 | 31.3 |
|---|---|---|---|---|---|---|
| 6 | Sterility | | | | A | A |
| | Phytotoxicity | | | | A | B |
| 7 | Sterility | A | A | A | | |
| | Phytotoxicity | B | B | B | | |
| 19 | Sterility | | | | A | A |
| | Phytotoxicity | | | | A | B |
| *Reference compound | Sterility | | C | B | A | |
| | Phytotoxicity | | A | A | C | |

* Potassium 1-(4-chlorophenyl)-1,4-dihydro-4-oxo-5-fluorocinnoline-3-carboxylate (the compound disclosed in EP-A- 197226)

Test Example 4

[Male sterility and female fertility test of wheat]

Wheat were grown according to the same method as in Test Example 1.

A designed amount of the test compounds formulated in an emulsifiable concentrate or a liquid formulation was diluted with water containing a spreading agent, and the dilution was sprayed over the foliage of the test plants by means of a small hand sprayer at a spray volume of 10 liters per are once to a pot at 13 days before the first heading time of the test plants.

Two pots were used per one experimental plot. One for an observation of the sterility of the test plants was kept free. The other for the female fertility was artificially pollinated with pollens obtained from the heads of untreated plants.

After the heading and flowering, artificial pollination was carried out as to 4 heads per pot of the pots which appeared to be sterile, using pollens obtained from the heads of untreated plants.

After ripening, there were harvested 4 heads per pot and each of spikelets and seeds thereof was counted.

The sterility rate and the fertility rate were calculated according to the following expression:

Sterility rate (%) = (1 - B/A) x 100
Fertility rate (%) = B/A x 100

A: the number of seeds per spikelet of an untreated plant
B: the number of seeds per spikelet of a treated plant

The results are shown in Table 8. In Table 8, "Sterility" is the sterility rate of no artificial pollination heads, and "Fertility" is the fertility rate of artificial pollination heads.

Table 8

| Compound No. | Dosage (g/ha) | Sterility (%) | Fertility (%) |
|---|---|---|---|
| 2 | 31.3 | 100 | 30.9 |
| 39 | 500 | 100 | 42.3 |
| 41 | 500 | 100 | 34.0 |
| Untreated control | | | |
| hand emasculated, not artificial pollinated | | | 0 |
| hand emasculated, artificial pollinated | | | 52.6 |

Test Example 5

[Sterility test of rice plant]

Plastic pots (volume: 200 mℓ) were filled with artificial soil mix and seeds of rice were sowed therein and grown under the same conditions as in Test Example 1.

Pots were flooded and then the test compounds were sprayed over the foliage of the test plants according to the same methods as in Test Example 1 once to a pot at 15 - 16 days before the first heading time of the test plants.

After ripening, there were harvested 4 heads per pot and glumous flower and seeds were counted.

The sterility rate was calculated according to the following expression:

Sterility rate (%) = (1 - B/A) x 100

A: the number of seeds per glumous flower of an untreated plant
B: the number of seeds per glumous flower of a treated plant

The test was carried out in one pot per treatment.

The results are shown in Table 9.

Table 9

| Compound No. | Dosage (g/ha) | Sterility rate (%) |
|---|---|---|
| 4 | 125 | 100 |
| 5 | 500 | 100 |
| 7 | 31.3 | 100 |
| 8 | 31.3 | 100 |
| 9 | 500 | 100 |
| 10 | 31.3 | 100 |
| 11 | 125 | 100 |
| 12 | 500 | 100 |
| 14 | 125 | 100 |
| 15 | 125 | 100 |
| 16 | 125 | 100 |
| 17 | 125 | 100 |
| 24 | 500 | 100 |
| 25 | 500 | 100 |
| 26 | 500 | 100 |
| 28 | 125 | 100 |
| 29 | 500 | 100 |
| 30 | 500 | 100 |
| 31 | 31.3 | 100 |
| 32 | 125 | 96.4 |
| 33 | 125 | 100 |
| 43 | 2000 | 100 |

Test Example 6

[Sterility test of rice plant]

Plastic pots (volume: 200 mℓ) were filled with artificial soil mix and seeds of rice were sowed therein and grown under the same conditions as in Test Example 1.

Pots were flooded and then the test compounds were sprayed over the foliage of the test plants according to the same methods as in Test Example 1 three times to the same pot, i.e. 23 days before, 15 days before and 9 days before the first heading time of the test plants.

After ripening, there were harvested 4 heads per pot and glumous flower and seeds were counted.

The sterility rate was calculated according to the same method as in Test Example 5.

The test was carried out in one pot per treatment. The results are shown in Table 10.

Table 10

| Compound No. | Dosage (g/ha) | Sterility rate (%) |
|---|---|---|
| 1 | 31.3 | 100 |
| 2 | 31.3 | 100 |
| 23 | 31.3 | 100 |
| 27 | 7.8 | 100 |
| 38 | 125 | 92.5 |
| 42 | 125 | 93.0 |
| 61 | 125 | 100 |

Test Example 7

[Male sterility and female fertility test of rice plant]

Paddy field soil was filled in 1/5000 are Wagner's pots. After creating the state of paddy field by flooding, rice plants of 17 days after sowing were transplanted and cultivated in the open air.

A designed amount of the test compounds formulated in a liquid formulation was diluted with water containing a spreading agent, and the dilution was sprayed over the foliage of the test plants by means of a small hand sprayer at a spray volume of 10 liters per are at 14 days before the heading time of the test plants.

Before the flowering, the heads of rice plants employed in the male sterility test were bagged.

As to rice plants employed in the female fertility test, on the flowering time, a hot water emasculation was carried out. All non-flowering glumous flowers were removed. As to flowering glumous flowers, the upper one-third of glumes thereof were cut off. After an artificial pollination was carried out with the pollen of rice plants which were not treated with the male sterilant, a bagging was carried out.

After ripening, there were harvested 4 heads per pot and glumous flower and seeds were counted.

The percentage of ripening was calculated according to the following expression:

$$\text{Percentage of ripening (\%)} = \frac{\text{the number of seeds of a head}}{\text{the number of glumous flower of a head}} \times 100$$

The test was carried out in 6 pots per treatment (3 pots for male sterility test and 3 pots for female fertility test).

The result of male sterility test is shown in Table 11 and the result of female fertility test is shown in Table 12.

Table 11

| Compound No. | Dosage (g/ha) | Percentage of ripening (%) |
|---|---|---|
| 25 | 400 | 0 |
| 32 | 400 | 0 |
| Untreated control*1 | 0 | 95.4 |

*1 no hot water emasculation and no artificial pollination

Table 12

| Compound No. | Dosage (g/ha) | Percentage of ripening (%) |
|---|---|---|
| 25 | 400 | 35.4 |
| 32 | 400 | 20.1 |
| Untreated control*2 | 0 | 0 |

*2 hot water emasculation was carried out but no artificial pollination

Test Example 8

[Sterility and female fertility test of morningglory]

Plastic pots (volume: 200 mℓ) were filled with plow-field soil and seeds of morningglory were sowed therein and grown under the same conditions as in Test Example 1 for 7 days. After that, there was carried out a short day treatment (22°C, day length of 8 hours) in a growth chamber for 14 days. After the short day treatment, the test plant was replaced under the same conditions as in Test Example 1.

A designed amount of the test compounds was sprayed over the foliage of the test plants according to the same methods as in Test Example 1 three times, i.e. 21, 28 and 35 days after sowing to the same pot. The test was carried out in two pots per treatment.

After flowering, six flowers per treatment were observed visually and rated with the following index.

Effect to the anther

A: No anther dehiscence

B: Anther dehiscence, few pollen number

C: Anther dehiscence, normal pollen number

Phytotoxicity

A: No or slight phytotoxicity

B: Phytotoxic, but the pistil is normal

C: Phytotoxic to the pistil

Artificial pollination was carried out to the flowers of which the effect to the anther was index A and the flowers of untreated control. The anther of the untreated control was hand-emasculated before flowering.

After seed set, the number of seed set flowers was counted and the fertility rate was calculated according to the following expression:

$$\text{Fertility rate (\%)} = \frac{\text{the number of seed set flowers}}{\text{the number of artificial pollinated flowers}} \times 100$$

The results are shown in Table 13.

Table 13

| Compound No. | Dosage (g/ha) | Effect to the anther | Phytotoxicity | Fertility rate (%) |
|---|---|---|---|---|
| 5 | 31.3 | A: 6 flowers | A: 6 flowers | 67 |
| 15 | 125 | A: 6 flowers | A: 6 flowers | 100 |
| Untreated control | 0 | C: 6 flowers | A: 6 flowers | 67 |

Test Example 9

[Sterility and female fertility test of morningglory]

Plastic pots (volume: 200 mℓ) were filled with plow-field soil and seeds of morningglory were sowed therein and grown under the same conditions as in Test Example 1 for 7 days. After that, there was carried out a short day treatment (22°C, day length of 8 hours) in a growth chamber for 14 days. After the short day treatment, the test plant was replaced under the same conditions as in Test Example 1.

A designed amount of the test compounds was sprayed over the foliage of the test plants according to the same methods as in Test Example 1 three times, i.e. 21, 28 and 35 days after sowing to the same pot. The test was carried out in two pots per treatment.

After flowering, six flowers per treatment were observed visually and rated with the index according to the same method as in Test Example 8.

Artificial pollination was carried out to the flowers of which the effect to the anther was index A and the flowers of untreated control. The anther of the untreated control was hand-emasculated before flowering.

After seed set, the number of seed set flowers was counted and the fertility rate was calculated according to the same method as in Test Example 8.

The results are shown in Table 14.

Table 14

| Compound No. | Dosage (g/ha) | Effect to the anther | Phytotoxicity | Fertility rate (%) |
|---|---|---|---|---|
| 53 | 2000 | A: 4 flowers<br>B: 2 flowers | A: 6 flowers | 100 |
| Untreated control | 0 | C: 6 flowers | A: 6 flowers | 100 |

In addition to the ingredients used in the Examples, Formulation Examples and Test Examples, other ingredients can be used in the Examples, Formulation Examples and Test Examples as set forth in the specification to obtain substantially the same results.

## Claims

**Claims for the following Contracting States : CH, DE, FR, GB, IT, LI, NL**

**1.** A plant male sterilant, which comprises as an active ingredient an effective amount of a compound having the general formula (I):

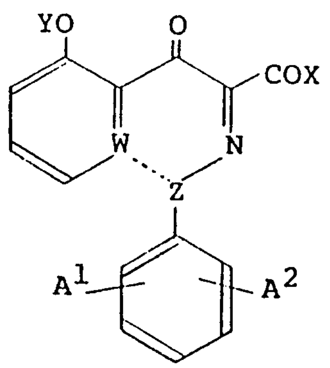

(I)

wherein:

X is $-OH$, $-O^{-}M^{+}$, $-OR^{I}$ or

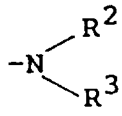

wherein $M^{+}$ is an alkali metal cation, an alkaline earth metal cation or

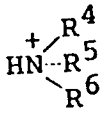

,

in which $R^4$, $R^5$ and $R^6$ are the same or different and each is a hydrogen atom, a $C_1-C_6$-alkyl group, a $C_3-C_4$-

alkenyl group, a $C_3-C_4$-alkynyl group, a $C_3-C_8$-cycloalkyl group, a benzyl group or a phenyl group; $R^1$ is a $C_1-C_9$-alkyl group, a $C_3-C_6$-alkenyl group, a $C_3-C_4$-alkynyl group, a $C_1-C_3$-alkoxy($C_1-C_4$)-alkyl group, a $C_1-C_3$-mono- or poly-haloalkyl group, a $C_3-C_8$-cycloalkyl group, a benzyl group or a phenyl group; and $R^2$ and $R^3$ are the same or different and each is a hydrogen atom, a $C_1-C_6$-alkyl group, a $C_3-C_4$-alkenyl group, a $C_3-C_4$-alkynyl group, a $C_3-C_8$-cycloalkyl group, a benzyl group in which at most two of hydrogen atoms at the $\alpha$-position thereof may be substituted by methyl group, a $C_2-C_3$-hydroxyalkyl group or a phenyl group in which at most three of hydrogen atoms thereof may be substituted by the same or different $C_1-C_2$-alkyl group or halogen atom;

Y is a $C_1-C_4$-mono- or polyhaloalkyl group;

$A^1$ and $A^2$ are the same or different and each is a hydrogen atom, a fluorine atom, a chlorine atom, a bromine atom, a trihalomethyl group, a $C_1-C_2$-alkylthio group or a $C_1-C_2$-haloalkoxy group; and

W and Z are C-F and N-H respectively with non-bonding, or are taken together to form C-N, provided that X is $OR^1$ in case that W and Z are C-F and N-H respectively,

and an inert carrier or diluent.

**2.** The plant male sterilant of Claim 1, in which W and Z are taken together to form C-N.

**3.** The plant male sterilant of Claim 2, in which X is $-OH$, $-O^-M^+$ or $-OR^1$.

**4.** The plant male sterilant of Claim 3, in which $A^1$ is a fluorine atom, a chlorine atom, a bromine atom, a trifluoromethyl group, a difluoromethoxy group or a trifluoromethoxy group and $A^2$ is a hydrogen atom or a fluorine atom.

**5.** The plant male sterilant of Claim 4, in which Y is a $C_1-C_2$-polyfluoroalkyl group.

**6.** The plant male sterilant of Claim 5, in which Y is a difluoromethyl group.

**7.** The plant male sterilant of Claim 5, in which Y is a trifluoromethyl group.

**8.** The plant male sterilant of Claim 5, in which Y is a 1,1,2,2-tetrafluoroethyl group.

**9.** The plant male sterilant of Claim 5, in which Y is a 2,2,2-trifluroethyl group.

**10.** The plant male sterilant of Claim 1, in which the active ingredient has the general formula (II):

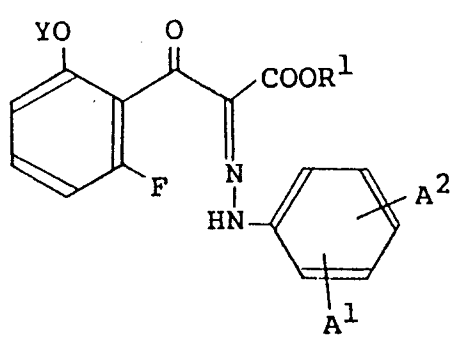

(II)

wherein

$R^1$ is a $C_1-C_9$-alkyl group, a $C_3-C_6$-alkenyl group, a $C_3-C_4$-alkynyl group, a $C_1-C_3$-alkoxy($C_1-C_4$)-alkyl group, a $C_1-C_3$-mono- or polyhaloalkyl group, a $C_3-C_8$-cycloalkyl group, a benzyl group or a phenyl group;

Y is a $C_1-C_4$-mono- or polyhaloalkyl group; and

$A^1$ and $A^2$ are the same or different and each is a hydrogen atom, a fluorine atom, a chlorine atom, a bromine atom, a trihalomethyl group, a $C_1-C_2$-alkylthio group or a $C_1-C_2$-haloalkoxy group.

**11.** The plant male sterilant of Claim 10, in which $A^1$ is a fluorine atom, a chlorine atom, a bromine atom, a trifluoromethyl group, a difluoromethoxy group or a trifluoromethoxy group and $A^2$ is a hydrogen atom or a fluorine atom.

**12.** The plant male sterilant of Claim 11, in which Y is a $C_1-C_2$-polyfluoroalkyl group.

**13.** A method for inducing male sterility in a plant, which comprises applying an effective amount of a compound having the formula (I) or (II) according to any of claims 1 to 12 and an inert carrier or diluent to the plant.

**14.** The method of Claim 13, in which the plant is wheat or rice.

**15.** A hydrazone derivative having the formula (II):

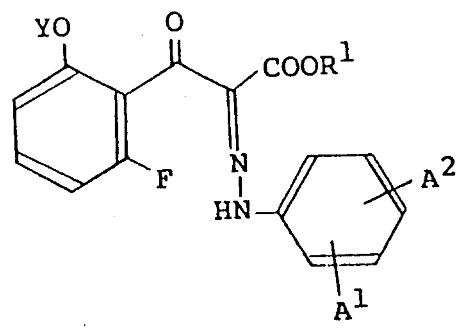

(II)

wherein

| | |
|---|---|
| $R^1$ | is a $C_1-C_9$-alkyl group, a $C_3-C_6$-alkenyl group, a $C_3-C_4$-alkynyl group, a $C_1-C_3$-alkoxy-($C_1-C_4$)-alkyl group, a $C_1-C_3$-mono- or polyhaloalkyl group, a $C_3-C_8$-cycloalkyl group, a benzyl group or a phenyl group; |
| Y | is a $C_1-C_4$ mono- or polyhaloalkyl group; and |
| $A^1$ and $A^2$ | are the same or different and each is a hydrogen atom, a fluorine atom, a chlorine atom, a bromine atom, a trihalomethyl group, a $C_1-C_2$-alkylthio group or a $C_1-C_2$-haloalkoxy group. |

**16.** The hydrazone derivative of Claim 15, in which $A^1$ is a fluorine atom, a chlorine atom, a bromine atom, a trifluoromethyl group, a difluoromethoxy group or a trifluoromethoxy group and $A^2$ is a hydrogen atom or a fluorine atom.

**17.** The hydrazone derivative of Claim 16, in which Y is a $C_1-C_2$-polyfluoroalkyl group.

**Claims for following Contracting State: ES**

**1.** A plant male sterilant, which comprises as an active ingredient an effective amount of a compound having the general formula (I):

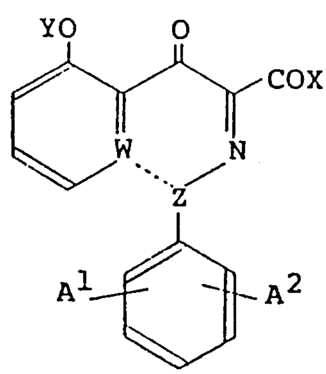

(I)

wherein:

X is $-OH$, $-O^-M^+$, $-OR^1$ or

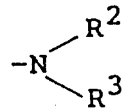

wherein $M^+$ is an alkali metal cation, an alkaline earth metal cation or

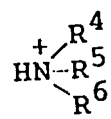

,

in which $R^4$, $R^5$ and $R^6$ are the same or different and each is a hydrogen atom, a $C_1-C_6$-alkyl group, a $C_3-C_4$-alkenyl group, a $C_3-C_4$-alkynyl group, a $C_3-C_8$-cycloalkyl group, a benzyl group or a phenyl group; $R^1$ is a $C_1-C_9$-alkyl group, a $C_3-C_6$-alkenyl group, a $C_3-C_4$-alkynyl group, a $C_1-C_3$-alkoxy($C_1-C_4$)-alkyl group, a $C_1-C_3$-mono- or poly-haloalkyl group, a $C_3-C_8$-cycloalkyl group, a benzyl group or a phenyl group; and $R^2$ and $R^3$ are the same or different and each is a hydrogen atom, a $C_1-C_6$-alkyl group, a $C_3-C_4$-alkenyl group, a $C_3-C_4$-alkynyl group, a $C_3-C_8$-cycloalkyl group, a benzyl group in which at most two of hydrogen atoms at the $\alpha$-position thereof may be substituted by methyl group, a $C_2-C_3$-hydroxyalkyl group or a phenyl group in which at most three of hydrogen atoms thereof may be substituted by the same or different $C_1-C_2$-alkyl group or halogen atom;

Y is a $C_1-C_4$-mono- or polyhaloalkyl group;

$A^1$ and $A^2$ are the same or different and each is a hydrogen atom, a fluorine atom, a chlorine atom, a bromine atom, a trihalomethyl group, a $C_1-C_2$-alkylthio group or a $C_1-C_2$-haloalkoxy group; and

W and Z are C-F and N-H respectively with non-bonding, or are taken together to form C-N, provided that X is $OR^1$ in case that W and Z are C-F and N-H respectively,

and an inert carrier or diluent.

**2.** The plant male sterilant of Claim 1, in which W and Z are taken together to form C-N.

**3.** The plant male sterilant of Claim 2, in which X is $-OH$, $-O^-M^+$ or $-OR^1$.

**4.** The plant male sterilant of Claim 3, in which $A^1$ is a fluorine atom, a chlorine atom, a bromine atom, a trifluoromethyl group, a difluoromethoxy group or a trifluoromethoxy group and $A^2$ is a hydrogen atom or a fluorine atom.

**5.** The plant male sterilant of Claim 4, in which Y is a $C_1-C_2$-polyfluoroalkyl group.

**6.** The plant male sterilant of Claim 5, in which Y is a difluoromethyl group.

**7.** The plant male sterilant of Claim 5, in which Y is a trifluoromethyl group.

**8.** The plant male sterilant of Claim 5, in which Y is a 1,1,2,2-tetrafluoroethyl group.

**9.** The plant male sterilant of Claim 5, in which Y is a 2,2,2-trifluroethyl group.

**10.** The plant male sterilant of Claim 1, in which the active ingredient has the general formula (II):

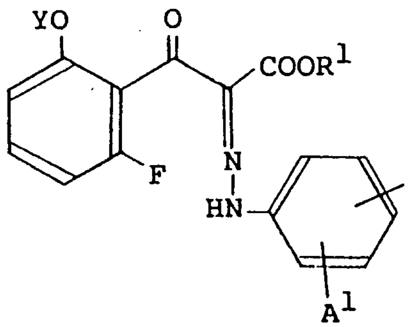

(II)

wherein

| | |
|---|---|
| $R^1$ | is a $C_1-C_9$-alkyl group, a $C_3-C_6$-alkenyl group, a $C_3-C_4$-alkynyl group, a $C_1-C_3$-alkoxy($C_1-C_4$)-alkyl group, a $C_1-C_3$-mono- or polyhaloalkyl group, a $C_3-C_8$-cycloalkyl group, a benzyl group or a phenyl group; |
| Y | is a $C_1-C_4$-mono- or polyhaloalkyl group; and |
| $A^1$ and $A^2$ | are the same or different and each is a hydrogen atom, a fluorine atom, a chlorine atom, a bromine atom, a trihalomethyl group, a $C_1-C_2$-alkylthio group or a $C_1-C_2$-haloalkoxy group. |

**11.** The plant male sterilant of Claim 10, in which $A^1$ is a fluorine atom, a chlorine atom, a bromine atom, a trifluoromethyl group, a difluoromethoxy group or a trifluoromethoxy group and $A^2$ is a hydrogen atom or a fluorine atom.

**12.** The plant male sterilant of Claim 11, in which Y is a $C_1-C_2$-polyfluoroalkyl group.

**13.** A method for inducing male sterility in a plant, which comprises applying an effective amount of a compound having the formula (I) or (II) according to any of claims 1 to 12 and an inert carrier or diluent to the plant.

**14.** The method of Claim 13, in which the plant is wheat or rice.

**15.** A process for preparing a hydrazone derivative having the formula (II):

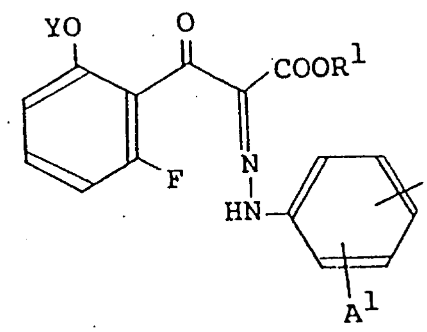

(II)

wherein

| | |
|---|---|
| $R^1$ | is a $C_1-C_9$-alkyl group, a $C_3-C_6$-alkenyl group, a $C_3-C_4$-alkynyl group, a $C_1-C_3$-alkoxy-($C_1-C_4$)-alkyl group, a $C_1-C_3$-mono- or polyhaloalkyl group, a $C_3-C_8$-cycloalkyl group, a benzyl group or a phenyl group; |
| Y | is a $C_1-C_4$ mono- or polyhaloalkyl group; and |
| $A^1$ and $A^2$ | are the same or different and each is a hydrogen atom, a fluorine atom, a chlorine atom, a bromine atom, a trihalomethyl group, a $C_1-C_2$-alkylthio group or a $C_1-C_2$-haloalkoxy group, |

characterized by

reacting a benzoylacetate derivative having the formula (III) :

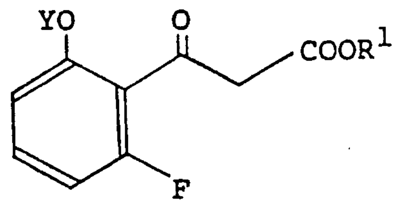

(III)

wherein $R^1$ and Y are as defined above, with a diazonium salt derivative having the formula (IV):

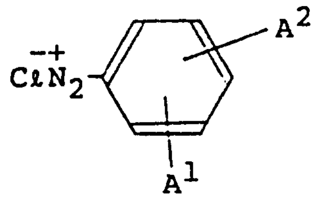

(IV)

wherein $A^1$ and $A^2$ are as defined above.

**16.** The process of Claim 15, in which $A^1$ is a fluorine atom, a chlorine atom, a bromine atom, a trifluoromethyl group, a difluoromethoxy group or a trifluoromethoxy group and $A^2$ is a hydrogen atom or a fluorine atom.

**17.** The process of Claim 16, in which Y is a $C_1-C_2$-polyfluoroalkyl group.

**18.** The process of any of claims 15 to 17, characterized by the fact, that the reaction is carried out in a solvent at a temperature of 0 to 50 °C for a period of 10 minutes to 10 hours.

**19.** The plant male sterilant of any Of claims 1 to 12 wherein the active ingredient is within a range of 1 to 80 % by weight.

**Patentansprüche**
**Patentansprüche für folgende Vertragsstaaten : CH, DE, FR, GB, IT, LI, NL**

**1.** Mittel zur Erzeugung einer männlichen Sterilität in Pflanzen, das als Wirkstoff eine effektive Menge einer Verbindung der allgemeinen Formel (I):

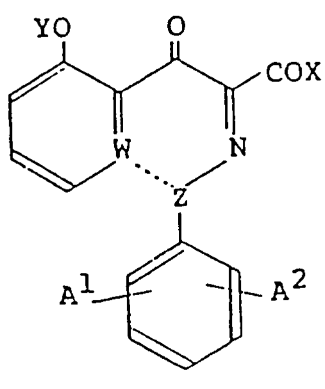

(I)

in der X $-OH$, $,O^-M^+$, $-OR^1$ oder

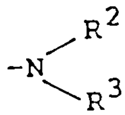

ist,
worin $M^+$ ein Alkalimetallkation, ein Erdalkalimetallkation oder

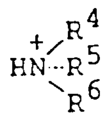

ist, worin $R^4$, $R^5$ und $R^6$ gleich oder verschieden sind und jedes ein Wasserstoffatom, eine $C_1 - C_6$-Alkylgruppe, eine $C_3 - C_4$-Alkenylgruppe, eine $C_3 - C_4$-Alkinylgruppe, eine $C_3 - C_8$-Cycloalkylgruppe, eine Benzylgruppe oder eine Phenylgruppe ist; $R^1$ eine $C_1 - C_9$-Alkylgruppe, eine $C_3 - C_6$-Alkenylgruppe, eine $C_3 - C_4$-Alkinylgruppe, eine $C_1 - C_3$-Alkoxy-$(C_1 - C_4)$-Alkylgruppe, eine $C_1 - C_3$-Mono- oder Polyhalogenalkylgruppe, eine $C_3 - C_8$-Cycloalkylgruppe, eine Benzylgruppe oder eine Phenylgruppe ist; und $R^2$ und $R^3$ gleich oder verschieden sind und jedes ein Wasserstoffatom, eine $C_1 - C_6$-Alkylgruppe, eine $C_3 - C_4$-Alkenylgruppe, eine $C_3 - C_4$-Alkinylgruppe, eine $C_3 - C_8$-Cycloalkylgruppe, eine Benzylgruppe, in der höchstens zwei der Wasserstoffatome in der $\alpha$-Position durch eine Methylgruppe substituiert sein können, eine $C_2 - C_3$-Hydroxyalkylgruppe oder eine Phenylgruppe, in der höchstens drei der Wasserstoffe durch gleiche oder verschiedene $C_1 - C_2$-Alkylgruppen oder Halogenatome substituiert sein können;
Y eine $C_1 - C_4$-Mono- oder Polyhalogenalkylgruppe ist;
$A^1$ und $A^2$ gleich oder verschieden sind und jedes ein Wasserstoffatom, ein Fluoratom, ein Chloratom, ein Bromatom, eine Trihalogenmethylgruppe, eine $C_1 - C_2$-Alkylthiogruppe oder eine $C_1 - C_2$-Halogenalkoxygruppe ist; und
W und Z C-F bzw. N-H ohne Bindung sind oder zusammen eine C-N-Bindung bilden, unter der Voraussetzung, daß X $OR^1$ ist für den Fall, daß W und Z C-F bzw. N-H sind,
und einen inerten Träger oder Verdünnungsmittel enthält.

**2.** Mittel zur Erzeugung einer männlichen Sterilität in Pflanzen nach Anspruch 1, in der W und Z gemeinsam eine C-N-Bindung bilden.

**3.** Mittel zur Erzeugung einer männlichen Sterilität in Pflanzen nach Anspruch 2, in der X $-OH$, $-O^-M^+$ oder $-OR^1$ ist.

**4.** Mittel zur Erzeugung einer männlichen Sterilität in Pflanzen nach Anspruch 3, in dem $A^1$ ein Fluoratom, ein Chloratom, ein Bromatom, eine Trifluormethylgruppe, eine Difluormethoxygruppe oder eine Trifluormethoxygruppe und $A^2$ ein Wasserstoffatom oder ein Fluoratom sind.

**5.** Mittel zur Erzeugung einer männlichen Sterilität in Pflanzen nach Anspruch 4, in dem Y eine $C_1 - C_2$-Polyfluoralkylgruppe ist.

**6.** Mittel zur Erzeugung einer männlichen Sterilität in Pflanzen nach Anspruch 5, in dem Y eine Difluormethylgruppe ist.

**7.** Mittel zur Erzeugung einer männlichen Sterilität in Pflanzen nach Anspruch 5, in dem Y eine Trifluormethylgruppe ist.

**8.** Mittel zur Erzeugung einer männlichen Sterilität in Pflanzen nach Anspruch 5, in dem Y eine 1,1,2,2-Tetrafluorethylgruppe ist.

**9.** Mittel zur Erzeugung einer männlichen Sterilität in Pflanzen nach Anspruch 5, in dem Y eine 2,2,2-Trifluorethylgruppe ist.

**10.** Mittel zur Erzeugung einer männlichen Sterilität in Pflanzen nach Anspruch 1, in dem der Wirkstoff die allgemeine Formel (II) hat:

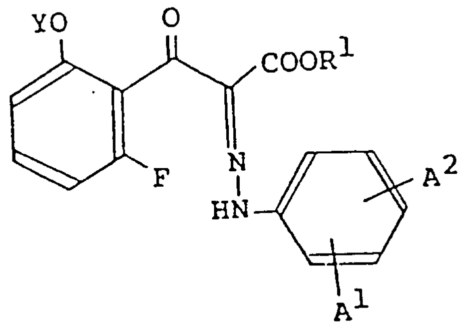

(II)

in der
$R^1$ eine $C_1 - C_9$-Alkylgruppe, eine $C_3 - C_6$-Alkenylgruppe, eine $C_3 - C_4$-Alkinylgruppe, eine $C_1 - C_3$-Alkoxy-$(C_1 - C_4)$-Alkylgruppe, eine $C_1 - C_3$-Mono- oder Polyhalogenalkylgruppe, eine $C_3 - C_8$-Cycloalkylgruppe, eine Benzylgruppe oder eine Phenylgruppe ist;
Y eine $C_1 - C_4$-Mono- oder Polyhalogenalkylgruppe ist und
$A^1$ und $A^2$ gleich oder verschieden sind und jedes ein Wasserstoffatom, ein Fluoratom, ein Chloratom, ein Bromatom, eine Trihalogenmethylgruppe, eine $C_1 - C_2$-Alkylthiogruppe oder eine $C_1 - C_2$-Halogenalkoxygruppe ist.

**11.** Mittel zur Erzeugung einer männlichen Sterilität in Pflanzen nach Anspruch 10, in dem $A^1$ ein Fluoratom, ein Chloratom, ein Bromatom, eine Trifluormethylgruppe, eine Difluormethoxygruppe oder eine Trifluormethoxygruppe und $A^2$ ein Wasserstoffatom oder ein Fluoratom sind.

**12.** Mittel zur Erzeugung einer männlichen Sterilität in Pflanzen nach Anspruch 11, in dem Y eine $C_1 - C_2$-Polyfluoralkylgruppe ist.

**13.** Verfahren zur Erzeugung einer männlichen Sterilität in Pflanzen, das die Anwendung einer effektiven Menge einer Verbindung der allgemeinen Formel (I) oder (II) nach einem der Ansprüche 1 bis 12 und eines inerten Trägers oder Verdünnungsmittels auf die Pflanze umfaßt.

**14.** Verfahren nach Anspruch 13, bei dem die Pflanze Weizen oder Reis ist.

**15.** Ein Hydrazonderivat der allgemeinen Formel (II):

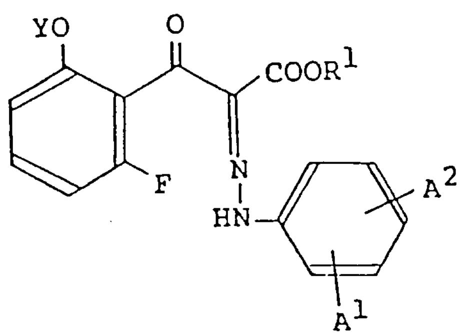

(II)

in der
$R^1$ eine $C_1 - C_9$-Alkylgruppe, eine $C_3 - C_6$-Alkenylgruppe, eine $C_3 - C_4$-Alkinylgruppe, eine $C_1 - C_3$-Alkoxy-$(C_1 - C_4)$-Alkylgruppe, eine $C_1 - C_3$-Mono- oder Polyhalogenalkylgruppe, eine $C_3 - C_8$-Cycloalkylgruppe, eine Benzylgruppe oder eine Phenylgruppe ist;
Y eine $C_1 - C_4$-Mono- oder Polyhalogenalkylgruppe ist und
$A^1$ und $A^2$ gleich oder verschieden sind und jedes ein Wasserstoffatom, ein Fluoratom, ein Chloratom, ein Bromatom, eine Trihalogenmethylgruppe, eine $C_1 - C_2$-Alkylthiogruppe oder eine $C_1 - C_2$-

Halogenalkoxygruppe ist.

**16.** Hydrazonderivat nach Anspruch 15, in dem $A^1$ ein Fluoratom, ein Chloratom, ein Bromatom, eine Trifluormethylgruppe, eine Difluormethoxygruppe oder eine Trifluormethoxygruppe und $A^2$ ein Wasserstoffatom oder ein Fluoratom sind.

**17.** Hydrazonderivat nach Anspruch 16, in dem Y eine $C_1 - C_2$-Polyfluoralkylgruppe ist.

**Patentansprüche für den folgenden Vertragsstaat : ES**

**1.** Mittel zur Erzeugung einer männlichen Sterilität in Pflanzen, das als Wirkstoff eine effektive Menge einer Verbindung der allgemeinen Formel (I):

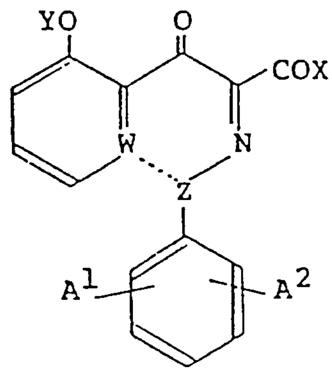

(I)

in der X $-OH$, $-O^-M^+$, $-OR^1$ oder

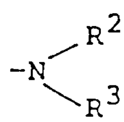

ist,
worin $M^+$ ein Alkalimetallkation, ein Erdalkalimetallkation oder

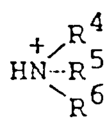

ist, worin $R^4$, $R^5$ und $R^6$ gleich oder verschieden sind
und jedes ein Wasserstoffatom, eine $C_1 - C_6$-Alkylgruppe, eine $C_3 - C_4$-Alkenylgruppe, eine $C_3 - C_4$-Alkinylgruppe, eine $C_3 - C_8$-Cycloalkylgruppe, eine Benzylgruppe oder eine Phenylgruppe ist; $R^1$ eine $C_1 - C_9$-Alkylgruppe, eine $C_3 - C_6$-Alkenylgruppe, eine $C_3 - C_4$-Alkinylgruppe, eine $C_1 - C_3$-Alkoxy-($C_1 - C_4$)-Alkylgruppe, eine $C_1 - C_3$-Mono- oder Polyhalogenalkylgruppe, eine $C_3 - C_8$-Cycloalkylgruppe, eine Benzylgruppe oder eine Phenylgruppe ist; und $R^2$ und $R^3$ gleich oder verschieden sind und jedes ein Wasserstoffatom, eine $C_1 - C_6$-Alkylgruppe, eine $C_3 - C_4$-Alkinylgruppe, eine $C_3 - C_4$-Alkinylgruppe, eine $C_3 - C_8$-Cycloalkylgruppe, eine Benzylgruppe, in der höchstens zwei der Wasserstoffatome in der $\alpha$-Position durch eine Methylgruppe substituiert sein können, eine $C_2 - C_3$-Hydroxyalkylgruppe oder eine Phenylgruppe, in der höchstens drei der Wasserstoffe durch gleiche oder verschiedene $C_1 - C_2$-Alkylgruppen oder Halogenatome substituiert sein können;
Y eine $C_1 - C_4$-Mono- oder Polyhalogenalkylgruppe ist;
$A^1$ und $A^2$ gleich oder verschieden sind und jedes ein Wasserstoffatom, ein Fluoratom, ein Chloratom, ein Bromatom, eine Trihalogenmethylgruppe, eine $C_1 - C_2$-Alkylthiogruppe oder eine $C_1 - C_2$-Halogenalkoxygruppe ist; und
W und Z C-F bzw. N-H ohne Bindung sind oder zusammen eine C-N-Bindung bilden, unter der

Voraussetzung, daß X $OR^1$ ist für den Fall, daß W und Z C − F bzw. N − H sind, und einen inerten Träger oder Verdünnungsmittel enthält.

**2.** Mittel zur Erzeugung einer männlichen Sterilität in Pflanzen nach Anspruch 1, in der W und Z gemeinsam eine C − N − Bindung bilden.

**3.** Mittel zur Erzeugung einer männlichen Sterilität in Pflanzen nach Anspruch 2, in der X − OH, $-O^-M^+$ oder $-OR^1$ ist.

**4.** Mittel zur Erzeugung einer männlichen Sterilität in Pflanzen nach Anspruch 3, in dem $A^1$ ein Fluoratom, ein Chloratom, ein Bromatom, eine Trifluormethylgruppe, eine Difluormethoxygruppe oder eine Trifluormethoxygruppe und $A^2$ ein Wasserstoffatom oder ein Fluoratom sind.

**5.** Mittel zur Erzeugung einer männlichen Sterilität in Pflanzen nach Anspruch 4, in dem Y eine $C_1 - C_2$ − Polyfluoralkylgruppe ist.

**6.** Mittel zur Erzeugung einer männlichen Sterilität in Pflanzen nach Anspruch 5, in dem Y eine Difluormethylgruppe ist.

**7.** Mittel zur Erzeugung einer männlichen Sterilität in Pflanzen nach Anspruch 5, in dem Y eine Trifluormethylgruppe ist.

**8.** Mittel zur Erzeugung einer männlichen Sterilität in Pflanzen nach Anspruch 5, in dem Y eine 1,1,2,2 − Tetrafluorethylgruppe ist.

**9.** Mittel zur Erzeugung einer männlichen Sterilität in Pflanzen nach Anspruch 5, in dem Y eine 2,2,2 − Trifluorethylgruppe ist.

**10.** Mittel zur Erzeugung einer männlichen Sterilität in Pflanzen nach Anspruch 1, in dem der Wirkstoff die allgemeine Formel (II) hat:

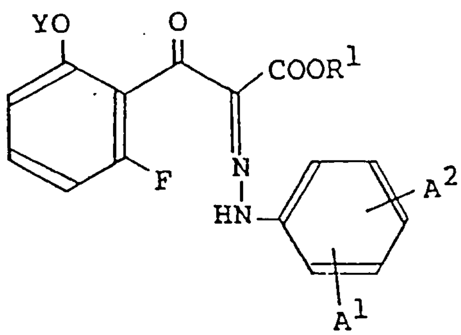

(II)

in der
$R^1$ eine $C_1 - C_9$ − Alkylgruppe, eine $C_3 - C_6$ − Alkenylgruppe, eine $C_3 - C_4$ − Alkinylgruppe, eine $C_1 - C_3$ − Alkoxy − ($C_1 - C_4$) − Alkylgruppe, eine $C_1 - C_3$ − Mono − oder Polyhalogenalkylgruppe, eine $C_3 - C_8$ − Cycloalkylgruppe, eine Benzylgruppe oder eine Phenylgruppe ist;
Y eine $C_1 - C_4$ − Mono − oder Polyhalogenalkylgruppe ist; und
$A^1$ und $A^2$ gleich oder verschieden sind und jedes ein Wasserstoffatom, ein Fluoratom, ein Chloratom, ein Bromatom, eine Trihalogenmethylgruppe, eine $C_1 - C_2$ − Alkylthiogruppe oder eine $C_1 - C_2$ − Halogenalkoxygruppe ist.

**11.** Mittel zur Erzeugung einer männlichen Sterilität in Pflanzen nach Anspruch 10, in dem $A^1$ ein Fluoratom, ein Chloratom, ein Bromatom, eine Trifluormethylgruppe, eine Difluormethoxygruppe oder eine Trifluormethoxygruppe und $A^2$ ein Wasserstoffatom oder ein Fluoratom sind.

**12.** Mittel zur Erzeugung einer männlichen Sterilität in Pflanzen nach Anspruch 11, in dem Y eine $C_1 - C_2$ − Polyfluoralkylgruppe ist.

**13.** Verfahren zur Erzeugung einer männlichen Sterilität in Pflanzen, das die Anwendung einer effektiven Menge einer Verbindung der allgemeinen Formel (I) oder (II) nach einem der Ansprüche 1 bis 12 und eines inerten Trägers oder Verdünnungsmittels auf die Pflanze umfaßt.

**14.** Verfahren nach Anspruch 13, bei dem die Pflanze Weizen oder Reis ist.

**15.** Ein Verfahren zur Herstellung eines Hydrazonderivats der allgemeinen Formel (II):

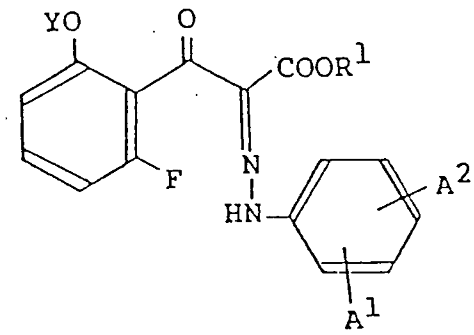

(II)

in der
$R^1$ eine $C_1-C_9$-Alkylgruppe, eine $C_3-C_6$-Alkenylgruppe, eine $C_3-C_4$-Alkinylgruppe, eine $C_1-C_3$-Alkoxy-$(C_1-C_4)$-Alkylgruppe, eine $C_1-C_3$-Mono- oder Polyhalogenalkylgruppe, eine $C_3-C_8$-Cycloalkylgruppe, eine Benzylgruppe oder eine Phenylgruppe ist;
Y eine $C_1-C_4$-Mono- oder Polyhalogenalkylgruppe ist; und
$A^1$ und $A^2$ gleich oder verschieden sind und jedes ein Wasserstoffatom, ein Fluoratom, ein Chloratom, ein Bromatom, eine Trihalogenmethylgruppe, eine $C_1-C_2$-Alkylthiogruppe oder eine $C_1-C_2$-Halogenalkoxygruppe ist, dadurch gekennzeichnet, daß ein Benzoylacetatderivat der allgemeinen Formel (III):

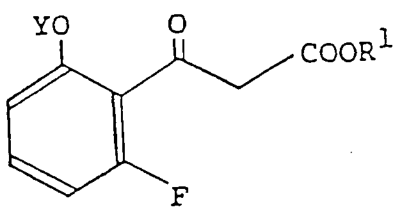

(III)

in der $R^1$ und Y der obigen Definition entsprechen, mit einem Diazoniumsalzderivat der allgemeinen Formel (IV):

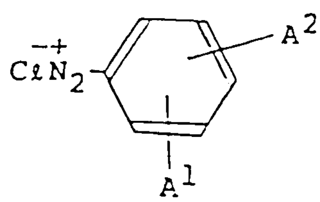

(IV)

in der $A^1$ und $A^2$ der obigen Definition entsprechen, umgesetzt wird.

**16.** Verfahren nach Anspruch 15, bei dem $A^1$ ein Fluoratom, ein Chloratom, ein Bromatom, eine Trifluormethylgruppe, eine Difluormethoxygruppe oder eine Trifluormethoxygruppe und $A^2$ ein Wasserstoffatom oder ein Fluoratom sind.

**17.** Verfahren nach Anspruch 16, in dem Y eine $C_1-C_2$-Polyfluoralkylgruppe ist.

**18.** Verfahren nach einem der Ansprüche 15 bis 17, dadurch gekennzeichnet, daß die Reaktion in einem Lösemittel bei einer Temperatur von 0 bis 50 °C über eine Zeit von 10 Minuten bis 10 Stunden durchgeführt wird.

**19.** Mittel zur Erzeugung einer männlichen Sterilität in Pflanzen nach einem der Ansprüche 1 bis 12, bei dem der Wirkstoff innerhalb eines Bereichs von 1 bis 80 Gew.-% vorliegt.

**Revendications**
**Revendications pour les Etats contractants suivants : DE, GB, FR, IT, NL, CH, LI**

**1.** Agent pour provoquer la stérilité mâle d'une plante, qui comprend comme principe actif une quantité efficace d'un composé ayant la formule générale:

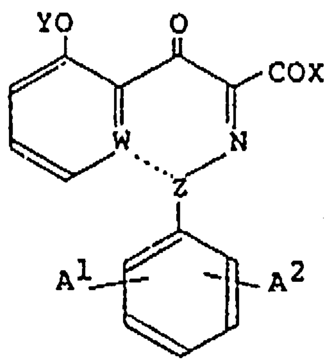

(I)

dans laquelle:

X représente $-OH$, $-O^{-}M^{+}$ $OR^1$ ou

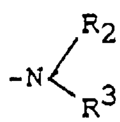

dans laquelle $M^+$ représente un cation de métal alcalin, un cation de métal alcalino-terreux ou

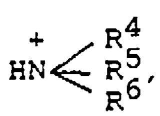

dans lequel $R^4$, $R^5$ et $R^6$ sont identiques ou différents et chacun représente un atome d'hydrogène, un groupement alkyle en $C_1$ à $C_6$, un groupement alcényle en $C_3$ à $C_4$, un groupement alcynyle en $C_3$ à $C_4$, un groupement cycloalkyle en $C_3$ à $C_8$, un groupement benzyle ou un groupement phényle, $R^1$ représente un groupement alkyle en $C_1$ à $C_9$, un groupement alcényle en $C_3$ à $C_6$, un groupement alcynyle en $C_3$ à $C_4$, un groupement alcoxy(en $C_1$ à $C_4$)-groupement alkyle(en $C_1$ à $C_4$), un groupement mono- ou polyhalogénoalkyle en $C_1$ à $C_3$, un groupement cycloalkyle en $C_3$ à $C_8$, un groupement benzyle ou un groupement phényle, et $R^2$ et $R^3$ sont identiques ou différents et chacun représente un atome d'hydrogène, un groupement alkyle en $C_1$ à $C_6$, un groupement alcényle en $C_3$ à $C_4$, un groupement alcynyle en $C_3$ à $C_4$, un groupement cycloalkyle en $C_3$ à $C_8$, un groupement benzyle dans lequel au plus deux de ses atomes d'hydrogène en position $\alpha$ peuvent être substitués par un groupement méthyle, un groupement hydroxyalkyle en $C_2$ à $C_3$ ou un groupement phényle dans lequel au plus trois de ses atomes d'hydrogène peuvent être substitués par un groupement alkyle en $C_1$ à $C_2$ identique ou différent, ou un atome d'halogène;

Y est un groupement mono- ou polyhalogénoalkyle en $C_1$ à $C_4$;

$A^1$ et $A^2$ sont identiques ou différents et chacun représente un atome d'hydrogène, un atome de fluor, un atome de chlore, un atome de brome, un groupement trihalogénométhyle, un groupement akylthio en $C_1$ à $C_2$ ou un groupement halogénoalkoxy en $C_1$ à $C_2$; et

W et Z représentent C−F et N−H respectivement sans liaison, ou sont pris ensemble pour former C−N, à condition que X soit $OR^1$ dans le cas où W et Z sont C−F et N−H respectivement;

et un diluant ou support inerte.

2. Agent pour provoquer la stérilité mâle d'une plante selon la revendication 1, dans lequel W et Z sont pris ensemble pour former C−N.

3. Agent pour provoquer la stérilité mâle d'une plante selon la revendication 2, dans lequel X représente $-OH$, $-O^-M^+$ ou $OR^1$.

4. Agent pour provoquer la stérilité mâle d'une plante selon la revendication 3, dans lequel $A^1$ représente un atome de fluor, un atome de chlore, un atome de brome, un groupement trifluorométhyle, un groupement difluorométhoxy ou un groupement trifluorométhoxy et $A^2$ représente un atome d'hydrogène ou un atome de fluor.

5. Agent pour provoquer la stérilité mâle d'une plante selon la revendication 4, dans lequel Y représente un groupement polyfluoroalkyle en $C_1$ à $C_2$.

6. Agent pour provoquer la stérilité mâle d'une plante selon la revendication 5, dans lequel Y représente un groupement difluorométhyle.

7. Agent pour provoquer la stérilité mâle d'une plante selon la revendication 5, dans lequel Y représente un groupement trifluorométhyle.

8. Agent pour provoquer la stérilité mâle d'une plante selon la revendication 5, dans lequel Y est un groupement 1,1,2,2−tétrafluoroéthyle.

9. Agent pour provoquer la stérilité mâle d'une plante selon la revendication 5, dans lequel Y est un groupement 2,2,2−trifluoroéthyle.

10. Agent pour provoquer la stérilité mâle d'une plante selon la revendication 1, dans lequel le principe actif a la formule générale (II):

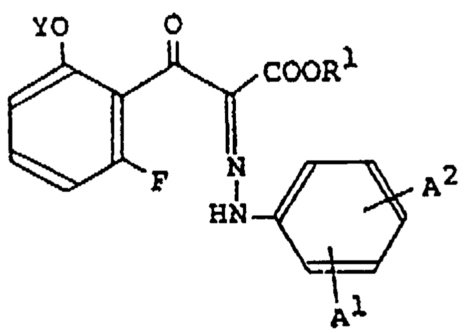

(II)

dans laquelle:

$R^1$ représente un groupement alkyle en $C_1$ à $C_9$, un groupement alcényle en $C_3$ à $C_6$, un groupement alcynyle en $C_3$ à $C_4$, un groupement alkoxy(en $C_1$ à $C_3$)−alkyle(en $C_1$ à $C_4$), un groupement mono ou polyhalogénoalkyle en $C_1$ à $C_3$, un groupement cycloalkyle en $C_3$ à $C_8$, un groupement benzyle ou un groupement phényle;

Y représente un groupement mono ou polyhalogénoalkyle en $C_1$ à $C_4$; et

$A^1$ et $A^2$ sont identiques ou différents et chacun représente un atome d'hydrogène, un atome de chlore, un atome de brome, un groupement trihalogénométhyle, un groupement alkythio en $C_1$ à $C_2$ ou un groupement halogénoalkoxy en $C_1$ à $C_2$.

**11.** Agent pour provoquer la stérilité mâle d'une plante selon la revendication 10, dans lequel $A^1$ représente un atome de fluor, un atome de chlore, un atome de brome, un groupement trifluorométhyle, un groupement difluorométhoxy ou un groupement trifluorométhoxy et $A^2$ représente un atome d'hydrogène ou un atome de fluor.

**12.** Agent pour provoquer la stérilité mâle d'une plante selon la revendication 11, dans lequel Y est un groupement polyfluoroalkyle en $C_1$ à $C_2$.

**13.** Procédé pour induire la stérilité mâle dans une plante, qui comprend l'application d'une quantité efficace d'un composé ayant la formule (I) ou (II) selon l'un quelconque des revendications 1 à 15 et d'un support ou diluant inerte sur la plante.

**14.** Procédé selon la revendication 13, dans lequel la plante est du blé ou du riz.

**15.** Dérivé hydrazone ayant la formule (II):

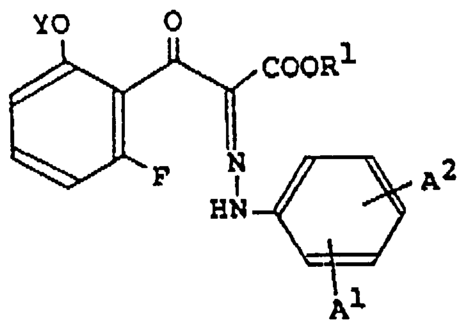

(II)

dans laquelle:

| | |
|---|---|
| $R^1$ | représente un groupement alkyle en $C_1$ à $C_9$, un groupement alcényle en $C_3$ à $C_6$, un groupement alcynyle en $C_3$ à $C_4$, un groupement alkoxy(en $C_1$ à $C_3$)-alkyle(en $C_1$ à $C_4$), un groupement mono ou polyhalogénoalkyle en $C_1$ à $C_3$, un groupement cycloalkyle en $C_3$ à $C_8$, un groupement benzyle ou un groupement phényle; |
| Y | représente un groupement mono ou polyhalogénoalkyle en $C_1$ à $C_4$; et |
| $A^1$ et $A^2$ | sont identiques ou différents et chacun représente un atome d'hydrogène, un atome de fluor, un atome de chlore, un atome de brome, un groupement trihalogénométhyle, un groupement alkythio en $C_1$ à $C_2$ ou un groupement halogénoalkoxy en $C_1$ à $C_2$. |

**16.** Dérivé d'hydrazone selon la revendication 15, dans lequel $A^1$ représente un atome de fluor, un atome de chlore, un atome de brome, un groupement trifluorométhyle, un groupement difluorométhoxy ou un groupement trifluorométhoxy et $A^2$ représente un atome d'hydrogène ou un atome de fluor.

**17.** Dérivé hydrazone selon la revendication 16, dans lequel Y représente un groupement polyfluoroalkyle en $C_1$ à $C_2$.

**Revendications pour l'Etat contractant suivant : ES**

**1.** Agent pour provoquer la stérilité mâle d'une plante, qui comprend comme principe actif une quantité efficace d'un composé ayant la formule générale:

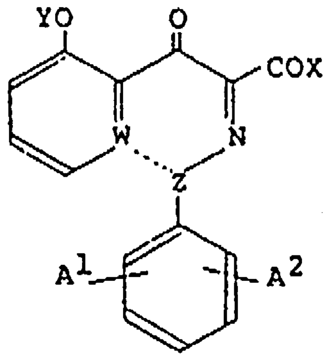

(I)

dans laquelle:

X représente $-OH$, $-O^-M^+$, $OR^1$ ou

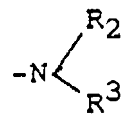

dans laquelle $M^+$ représente un cation de métal alcalin, un cation de métal alcalino-terreux ou

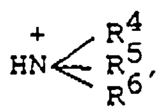

dans lequel $R^4$, $R^5$ et $R^6$ sont identiques ou différents et chacun représente un atome d'hydrogène, un groupement alkyle en $C_1$ à $C_6$, un groupement alcényle en $C_3$ à $C_4$, un groupement alcynyle en $C_3$ à $C_4$, un groupement cycloalkyle en $C_3$ à $C_8$, un groupement benzyle ou un groupement phényle, $R^1$ représente un groupement alkyle en $C_1$ à $C_9$, un groupement alcényle en $C_3$ à $C_6$, un groupement alcynyle en $C_3$ à $C_4$, un groupement alcoxy(en $C_1$ à $C_4$)-groupement alkyle(en $C_1$ à $C_4$), un groupement mono- ou polyhalogénoalkyle en $C_1$ à $C_3$, un groupement cycloalkyle en $C_3$ à $C_8$, un groupement benzyle ou un groupement pnényle, et $R^2$ et $R^3$ sont identiques ou différents et chacun représente un atome d'hydrogène, un groupement alkyle en $C_1$ à $C_6$, un groupement alcényle en $C_3$ à $C_4$, un groupement alcynyle en $C_3$ à $C_4$, un groupement cycloalkyle en $C_3$ à $C_8$, un groupement benzyle dans lequel au plus deux de ses atomes d'hydrogène en position $\alpha$ peuvent être substitués par un groupement méthyle, un groupement hydroxyalkyle en $C_2$ à $C_3$ ou un groupement phényle dans lequel au plus trois de ses atomes d'hydrogène peuvent être substitués par un groupement alkyle en $C_1$ à $C_2$ identique ou différent, ou un atome d'halogène;

Y est un groupement mono- ou polyhalogénoalkyle en $C_1$ à $C_4$;

$A^1$ et $A^2$ sont identiques ou différents et chacun représente un atome d'hydrogène, un atome de fluor, un atome de chlore, un atome de brome, un groupement trihalogénométhyle, un groupement akylthio en $C_1$ à $C_2$ ou un groupement halogénoalkoxy en $C_1$ à $C_2$; et

W et Z représentent C-F et N-H respectivement sans liaison, ou sont pris ensemble pour former C-N, à condition que X soit $OR^1$ dans le cas où W et Z sont C-F et N-H respectivement;

et un diluant ou support inerte.

**2.** Agent pour provoquer la stérilité mâle d'une plante selon la revendication 1, dans lequel W et Z sont pris ensemble pour former C-N.

3. Agent pour provoquer la stérilité mâle d'une plante selon la revendication 2, dans lequel X représente $-OH$, $-O^{-}M^{+}$ ou $OR^1$.

4. Agent pour provoquer la stérilité mâle d'une plante selon la revendication 3, dans lequel $A^1$ représente un atome de fluor, un atome de chlore, un atome de brome, un groupement trifluorométhyle, un groupement difluorométhoxy ou un groupement trifluorométhoxy et $A^2$ représente un atome d'hydrogène ou un atome de fluor.

5. Agent pour provoquer la stérilité mâle d'une plante selon la revendication 4, dans lequel Y représente un groupement polyfluoroalkyle en $C_1$ à $C_2$.

6. Agent pour provoquer la stérilité mâle d'une plante selon la revendlcation 5, dans lequel Y représente un groupement difluorométhyle.

7. Agent pour provoquer la stérilité mâle d'une plante selon la revendication 5, dans lequel Y représente un groupement trifluorométhyle.

8. Agent pour provoquer la stérilité mâle d'une plante selon la revendication 5, dans lequel Y est un groupement 1,1,2,2-tétrafluoroéthyle.

9. Agent pour provoquer la stérilité mâle d'une plante selon la revendication 5, dans lequel Y est un groupement 2,2,2-trifluoroéthyle.

10. Agent pour provoquer la stérilité mâle d'une plante selon la revendication 1, dans lequel le principe actif a la formule générale (II):

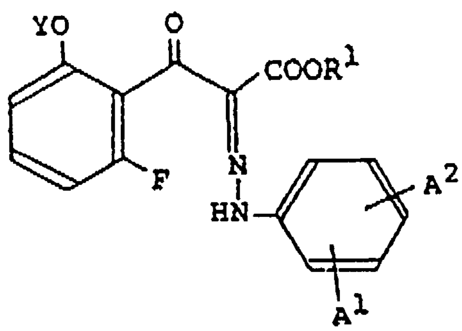

(II)

dans laquelle:

| | |
|---|---|
| $R^1$ | représente un groupement alkyle en $C_1$ à $C_9$, un groupement alcényle en $C_3$ à $C_6$, un groupement alcynyle en $C_3$ à $C_4$, un groupement alkoxy(en $C_1$ à $C_3$)-alkyle(en $C_1$ à $C_4$), un groupement mono ou polyhalogénoalkyle en $C_1$ à $C_3$, un groupement cycloalkyle en $C_3$ à $C_8$, un groupement benzyle ou un groupement phényle; |
| Y | représente un groupement mono ou polyhalogénoalkyle en $C_1$ à $C_4$; et |
| $A^1$ et $A^2$ | sont identiques ou différents et chacun représente un atome d'hydrogène, un atome de chlore, un atome de brome, un groupement trihalogénométhyle, un groupement alkythio en $C_1$ à $C_2$ ou un groupement halogénoalkoxy en $C_1$ à $C_2$. |

11. Agent pour provoquer la stérilité mâle d'une plante selon la revendication 10, dans lequel $A^1$ représente un atome de fluor, un atome de chlore, un atome de brome, un groupement trifluorométhyle, un groupement difluorométhoxy ou un groupement trifluorométhoxy et $A^2$ représente un atome d'hydrogène ou un atome de fluor.

12. Agent pour provoquer la stérilité mâle d'une plante selon la revendication 11, dans lequel Y est un groupement polyfluoroalkyle en $C_1$ à $C_2$.

13. Procédé pour induire la stérilité mâle dans une plante, qui comprend l'application d'une quantité efficace d'un composé ayant la formule (I) ou (II) selon l'un quelconque des revendications 1 à 15 et

d'un support ou diluant inerte sur la plante.

**14.** Procédé selon la revendication 13, dans lequel la plante est du blé ou du riz.

**15.** Procéde pour préparer un dérivé hydrazone ayant la formule (II):

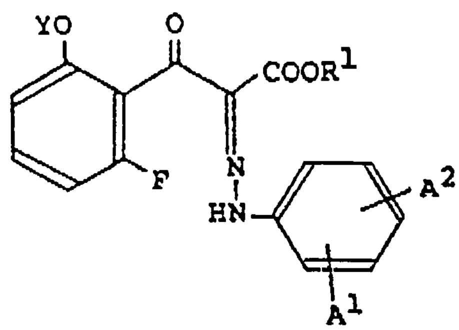

(II)

dans laquelle:

| | |
|---|---|
| $R^1$ | représente un groupement alkyle en $C_1$ à $C_9$, un groupement alcényle en $C_3$ à $C_6$, un groupement alcynyle en $C_3$ à $C_4$, un groupement alkoxy(en $C_1$ à $C_3$)-alkyle(en $C_1$ à $C_4$), un groupement mono ou polyhalogénoalkyle en $C_1$ à $C_3$, un groupement cycloalkyle en $C_3$ à $C_8$, un groupement benzyle ou un groupement phényle; |
| Y | représente un groupement mono ou polyhalogénoalkyle en $C_1$ à $C_4$; et |
| $A^1$ et $A^2$ | sont identiques ou différents et chacun représente un atome d'hydrogène, un atome de fluor, un atome de chlore, un atome de brome, un groupement trihalogénométhyle, un groupement alkythio en $C_1$ à $C_2$ ou un groupement halogénoalkoxy en $C_1$ à $C_2$, |

caractérisé par
réagir un dérivé d'acetate de benzoyle ayant la formule (III):

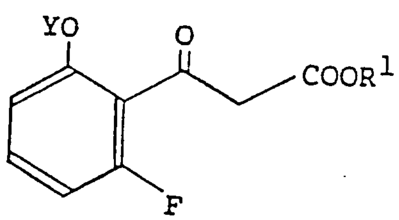

(III)

dans laquelle $R^1$ et Y sont définis comme ci-dessus, avec un dérivé d'un sel de diazonium ayant la formule (IV):

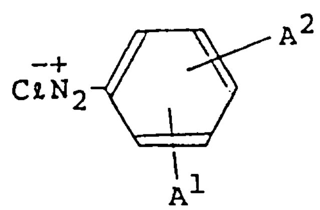

(IV)

dans laquelle $A^1$ et $A^2$ sont définis comme ci-dessus.

**16.** Procédé selon la revendication 15, dans lequel $A^1$ représente un atome de fluor, un atome de chlore, un atome de brome, un groupement trifluorométhyle, un groupement difluorométhoxy ou un groupement trifluorométhoxy et $A^2$ représente un atome d'hydrogène ou un atome de fluor.

**17.** Procédé selon la revendication 16, dans lequel Y représente un groupement polyfluoroalkyle en $C_1$ à $C_2$.

**18.** Procédé selon l'un des revendications 15 à 17, caractérisé en ce que la réaction se performe dans un solvant à une température de 0 à 50 °C pour une période de 10 minutes à 10 heures.

**19.** Agent pour provoquer la stérilité mâle d'une plante selon l'un des revendications 1 à 12, dans lequel le principe actif est dans un régime de 1 à 80 % en poids.